# EUROPÄISCHE PATENTSCHRIFT

(11) **EP 1 144 623 B1**
(45) Veröffentlichungstag und Bekanntmachung des Hinweises auf die Patenterteilung: **28.08.2002**
(21) Anmeldenummer: 00910510.7
(22) Anmeldetag: 29.01.2000
(51) Int. Cl.: C12N 15/11, A61K 31/713

(54) **VERFAHREN UND MEDIKAMENT ZUR HEMMUNG DER EXPRESSION EINES VORGEGEBENEN GENS**
METHOD AND MEDICAMENT FOR INHIBITING THE EXPRESSION OF A DEFINED GENE
METHODE ET MEDICAMENT DESTINES A INHIBER L'EXPRESSION D'UN GENE DONNE

(30) Priorität: 30.01.1999 DE 19903713; 24.11.1999 DE 19956568
(43) Veröffentlichungstag der Anmeldung: 17.10.2001
(60) Teilanmeldung: 02003683.6
(73) Patentinhaber: Ribopharma AG, 95440 Bayreuth (DE)
(72) Erfinder: KREUTZER, Roland, 95466 Weidenberg (DE); LIMMER, Stephan, 95447 Bayreuth (DE)
(74) Vertreter: Gassner, Wolfgang, Dr.
(86) Internationale Anmeldenummer: DE0000244
(87) Internationale Veröffentlichungsnummer: WO00044895

(56) Entgegenhaltungen:
- WO-A-92/19732
- WO-A-98/05770
- WO-A-99/32619
- UHLMANN E ET AL: "ANTISENSE OLIGONUCLEOTIDES: A NEW THERAPEUTIC PRINCIPLE" CHEMICAL REVIEWS,US,AMERICAN CHEMICAL SOCIETY. EASTON, Bd. 90, Nr. 4, 1. Juni 1990 (1990-06-01), Seiten 543-584, XP000141412 ISSN: 0009-2665
- MADHUR K. ET AL.: "Antisense RNA : function and fate of duplex RNA in cells of higher eukaryotes." MICROBIOLOGY AND MOLECULAR BIOLOGY REVIEWS, Bd. 62, Dezember 1998 (1998-12), Seiten 1415-1434, XP000909741

## Beschreibung

Die Erfindung betrifft Verfahren nach dem Oberbegriff des Anspruchs 1. Sie betrifft ferner ein Medikament nach dem Oberbegriff des Anspruchs 32, einen Wirkstoff nach dem Oberbegriff des Anspruchs 63 sowie Verwendungen nach den Oberbegriffen der Ansprüche 81 und 114.

Ein solches Verfahren ist aus der nachveröffentlichten WO 99/32619 bekannt. Das bekannte Verfahren zielt auf die Hemmung der Expression von Genen in Zellen von Invertebraten ab. Dazu ist es erforderlich, daß das doppelsträngige Oligoribonukleotid eine zum Zielgen identische Sequenz mit einer Länge von mindestens 25 Basen aufweist. Zur Erzielung einer effizienten Hemmung ist eine Länge der identischen Sequenz von 300 bis 1000 Basenpaare erforderlich. Der Herstellungsaufwand eines solchen Qligoribonukleotids ist hoch.

Die DE 196 31 919 C2 beschreibt eine Anti-Sinn-RNA mit besonderen Sekundärstrukturen, wobei die Anti-Sinn-RNA in Form eines sie kodierenden Vektors vorliegt. Bei der Anti-Sinn-BNA handelt es sich um ein RNA-Molekül, das komplementär zu Bereichen der mRNA ist. Durch Bindung an diese Bereiche wird eine Hemmung der Genexpression bewirkt. Diese Hemmung kann insbesondere zur Diagnose und/oder Therapie von Erkrankungen, z.B. Tumorerkrankungen oder viralen Infektionen, eingesetzt werden. - Die Anti-Sinn-RNA muß nachteiligerweise in einer Menge in die Zelle eingebracht werden, die mindestens genauso groß wie die Menge der mRNA ist. Die Wirksamkeit der bekannten Anti-Sinn-Verfahren ist nicht besonders hoch.

Aus der US 5,712,257 ist ein Medikament bekannt, das fehlgepaarte doppelsträngige RNA (dsRNA) und biologisch aktive fehlgepaarte Bruchstücke von dsRNA in Form eines ternären Komplexes mit einem oberflächenaktiven Mittel enthält. Die dabei verwendete dsRNA besteht aus synthetisch hergestellten Nukleinsäureeinzelsträngen ohne definierte Basensequenz. Die Einzelstränge gehen nicht-reguläre, sogenannte "Nicht-Watson-Crick"-Basenpaarungen miteinander ein, so daß fehlgepaarte Doppelstränge gebildet werden. Die bekannte dsRNA dient zur Hemmung der Vermehrung von Retroviren, wie HIV. Die Vermehrung des Virus kann gehemmt werden, wenn nicht-sequenzspezifische dsRNA in die Zellen eingebracht wird. Es kommt dabei zu einer Induktion von Interferon, wodurch die Virusvermehrung gehemmt werden soll. Der hemmende Effekt bzw. die Wirksamkeit dieses Verfahrens ist gering.

Aus Fire, A. et.al, NATURE, Vol. 391, pp. 806 ist es bekannt, daß dsRNA, deren einer Strang abschnittsweise komplementär zu einem zu hemmenden Gen eines Fadenwurms ist, die Expression dieses Gens mit einer hohen Wirksamkeit hemmt. Es wird die Auffassung vertreten, daß die besondere Wirksamkeit der verwendeten dsRNA in Zellen des Fadenwurms nicht auf dem Anti-Sinn-Prinzip beruht, sondern möglicherweise auf katalytische Eigenschaften der dsRNA bzw. durch sie induzierte Enzyme zurückzuführen ist. - Über die Wirksamkeit spezifischer dsRNA in bezug auf die Hemmung der Genexpression, insbesondere in Säugerzellen und humanen Zellen, ist in diesem Artikel nichts ausgesagt.

Die WO 92/19732 betrifft Anti-Sinn-Oligonukleotide und deren Verwendung. Die einzelsträngigen Oligonukleotide sind zum Schutz gegen enzymatischen Abbau durch Exonukleasen rückgefaltet oder topologisch geschlossen. Ihre Wirkung beruht insbesondere auf der Blockade der Expression von Zielgenen durch Tripel-Helixbindung.

Die WO 98/05770 betrifft eine Anti-Sinn-RNA. Die Anti-Sinn-RNA weist einen doppelsträngigen Bereich auf. Dieser Bereich ist jedoch nicht komplementär zum Zielgen. Beim doppelsträngigen Bereich handelt es sich um eine durch eine Poly-GCGC ... - sequenz erzeugte selbstkomplementäre Faltung, welche der Stabilisierung des Moleküls dient.

Ulmann et al., 2377 Chemical Review, 90 (1990) June, No. 4, Washington DC, US gibt einen Überblick über die Wirkungsweise von Anti-Sinn-Oligonukleotiden.

Aufgabe der vorliegenden Erfindung ist es, die Nachteile nach dem Stand der Technik zu beseitigen. Es soll insbesondere ein möglichst effizientes Verfahren, Medikament bzw. Wirkstoff und eine möglichst effiziente Verwendung zur Herstellung eines Medikaments bzw. Wirkstoffs angegeben werden, mit dem/der eine besonders wirksame Hemmung der Expression eines vorgegebenen Zielgens bewirkbar ist.

Diese Aufgabe wird durch die Merkmale der Ansprüche 1, 32, 63, 81 und 114 gelöst. Vorteilhafte Ausgestaltungen ergeben sich aus den Ansprüchen 2 bis 31, 33 bis 62, 882 bis 113 und 115 bis 125.

Die Aufgabe wird gelöst u.a. durch die Merkmale Anspruchs 1.

Es hat sich überraschenderweise gezeigt, daß bereits bei einer Länge des komplementären Bereichs von weniger als 25 aufeinanderfolgenden Nukleotidpaaren eine wirksame Hemmung der Expression des Zielgens erreicht werden kann. Entsprechende Oligoribonukleotide können mit geringerem Herstellungsaufwand bereitgestellt werden.

Insbesondere dsRNA mit einer Länge von mehr als 50 Nukleotidpaaren induziert in Säugerzellen und humanen Zellen bestimmte zelluläre Mechanismen, z.B. die dsRNA-abhängige Proteinkinase oder das 2-5A-System. Das führt zum Verschwinden des durch die eine definierte Sequenz aufweisende dsRNA vermittelten Interferenzeffektes. Dadurch wird die Proteinbiosynthese in der Zelle blockiert. Insbesondere dieser Nachteil wird durch die vorliegende Erfindung beseitigt.

Weiterhin ist die Aufnahme von dsRNA mit kurzer Kettenlänge in die Zelle bzw. in den Zellkern gegenüber längerkettigen dsRNAs deutlich erleichtert.

Es hat sich als vorteilhaft erwiesen, daß die dsRNA in micellare Strukturen, vorzugsweise in Liposomen, vorliegt. Die dsRNA kann gleichfalls in virale natürliche Kapside oder in auf chemischem oder enzymatischem Weg hergestellte künstliche Kapside oder davon abgeleitete Strukturen eingeschlossen sein. - Die vorgenannten Merkmale ermöglichen ein Einschleusen der dsRNA in vorgegebene Zielzellen.

Das zu hemmende Gen wird zweckmäßigerweise in eukaryontischen Zellen exprimiert. Das Zielgen kann aus der folgenden Gruppe ausgewählt sein: Onkogen, Cytokin-Gen, Id-Protein-Gen, Entwicklungsgen, Priongen. Es kann auch in pathogenen Organismen, vorzugsweise in Plasmodien, exprimiert werden. Es kann Bestandteil eines, vorzugsweise humanpathogenen, Virus oder Viroids sein. - Das vorgeschlagene Verfahrenermöglicht die Herstellung von Mitteln zur Therapie genetisch gesteuerter Krankheiten, z.B. Krebs, viraler Erkrankungen oder Morbus Alzheimer.

Das Virus oder Viroid kann auch ein tier- oder planzenpathogenes Virus oder Viroid sein. In diesem Fall erlaubt das erfindungsgemäße Verfahren auch die Bereitstellung von Mitteln zur Behandlung von Tier- oder Pflanzenkrankheiten.

Nach einem weiteren Ausgestaltungsmerkmal ist die dsRNA abschnittsweise doppelsträngig ausgebildet. Die Enden der dsRNA können modifiziert werden, um einem Abbau in der Zelle oder einer Dissoziation in die Einzelstränge entgegenzuwirken. Eine Dissoziation tritt insbesondere bei Verwendung niedriger Konzentrationen oder kurzer Kettenlängen auf. Zur besonders wirksamen Hemmung der Dissoziation kann der durch die Nukleotidpaare bewirkte Zusammenhalt der doppelsträngigen Struktur durch mindestens eine, vorzugsweise zwei, weitere chemische Verknüpfung/en erhöht werden. - Eine erfindungsgemäße dsRNA, deren Dissoziation vermindert ist, weist eine höhere Stabilität gegen enzymatischen und chemischen Abbau in der Zelle bzw. im Organismus auf.

Die chemische Verknüpfung wird zweckmäßigerweise durch eine kovalente oder ionische Bindung, eine Wasserstoffbrückenbindung, hydrophobe Wechselwirkungen, vorzugsweise van-der-Waalsoder Stapelungswechselwirkungen, oder durch Metall-Ionenkoordination gebildet. Sie kann nach einem besonders vorteilhaften Ausgestaltungsmerkmal an mindestens einem, vorzugsweise an beiden, Ende/n hergestellt werden.

Es hat sich weiter als vorteilhaft erwiesen, daß die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Polyethylenglycol-Ketten sind. Die chemische Verknüpfung kann auch durch in der doppelsträngigen Struktur anstelle von Purinen benutzte Purinanaloga gebildet werden. Von Vorteil ist es ferner, daß die chemische Verknüpfung durch in der doppelsträngigen Struktur eingeführte Azabenzoleinheiten gebildet wird. Sie kann außerdem durch in der doppelsträngigen Struktur anstelle von Nukleotiden benutzte verzweigte Nukleotidanaloga gebildet werden.

Es hat sich als zweckmäßig erwiesen, daß zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxylbenzoyl)-cystamin; 4-Thiouracil; Psoralen. Ferner kann die chemische Verknüpfung durch an den Enden des doppelsträngigen Bereichs angebrachte Thiophosphoryl-Gruppen gebildet werden. Vorzugsweise wird die chemische Verknüpfung an den Enden des doppelsträngigen Bereichs durch Tripelhelix-Bindungen hergestellt.

Die chemische Verknüpfung kann zweckmäßigerweise durch ultraviolettes Licht induziert werden.

Die Nukleotide der dsRNA können modifiziert sein. Dies wirkt einer Aktivierung einer von doppelsträngiger RNA abhängigen Proteinkinase, PKR, in der Zelle entgegen. Vorteilhafterweise ist mindestens eine 2'-Hydroxylgruppe der Nukleotide der dsRNA in der doppelsträngigen Struktur durch eine chemische Gruppe, vorzugsweise eine 2'-Amino- oder eine 2'-Methylgruppe, ersetzt. Mindestens ein Nukleotid in mindestens einem Strang der doppelsträngigen Struktur kann auch ein sogenanntes "locked nucleotide" mit einem, vorzugsweise durch eine 2'-O, 4'-C-Methylenbrücke, chemisch modifizierten Zuckerring sein. Vorteilhafterweise sind mehrere Nukleotide "locked nucleotides".

Nach einer weiteren besonders vorteilhaften Ausgestaltung ist vorgesehen, daß die dsRNA an mindestens ein von einem Virus stammendes, davon abgeleitetes oder ein synthetisch hergestelltes virales Hüllprotein gebunden, damit assoziiert oder davon umgeben wird. Das Hüllprotein kann vom Polyomavirus abgeleitet sein. Es kann das Hüllprotein das Virus-Protein 1 (VP1) und/oder das Virus-Protein 2 (VP2) des Polyomavirus enthalten. Die Verwendung derartiger Hüllproteine ist z.B. aus der DE 196 18 797 A1 bekannt. - Die vorgenannten Merkmale erleichtert wesentlich das Einführen der dsRNA in die Zelle.

Vorzugsweise ist bei Bildung eines Kapsids oder kapsidartigen Gebildes aus dem Hüllprotein die eine Seite zum Inneren des Kapsids oder kapsidartigen Gebildes gewandt. Das gebildete Konstrukt ist besonders stabil.

Die dsRNA kann zum primären oder prozessierten RNA-Transkript des Zielgens komplementär sein.- Die Zelle kann eine Vertebratenzelle oder eine menschliche Zelle sein.

Es können mindestens zwei voneinander verschiedene dsRNAs in die Zelle eingeführt werden, wobei ein Strang jeder dsRMA zumindest abschnittsweise komplementär zu jeweils einem von mindestens zwei verschiedenen Zielgenen ist. Dadurch ist es möglich. gleichzeitig die Expression mindestens zwei verschiedener Zielgene zu hemmen. Um die Expression einer von doppelsträngiger RNA abhängigen Proteinkinase, PKR, in der-Zelle zu unterdrücken, ist eines der Zielgene vorteilhafterweise das PKR-Gen. Dadurch kann die PKR-Aktivität in der Zelle wirksam unterdrückt werden.

Nach Maßgabe der Erfindung wird die Aufgabe ferner durch ein Medikament mit den Merkmalen der Ansprüche 32 bzw. 63 gelöst. - Es hat sich überraschend gezeigt, daß eine solche dsRNA sich als Medikament zur Hemmung der Expression eines vorgegebenen Gens in Säugerzellen bzw. in einem pflanzenpathogenen Virus oder Viroids eignet. Die Hemmung wird im Vergleich zur Verwendung einzelsträngiger Oligoribonukleotide bereits bei Konzentrationen bewirkt, die um mindestens eine Größenordnung niedriger sind. Das erfindungsgemäße Medikament ist hoch wirksam. Es sind geringere Nebenwirkungen zu erwarten.

Nach weiterer Maßgabe der Erfindung wird die Aufgabe durch eine Verwendung eines Oligoribonukleotids mit den Merkmalen des des Anspruchs 81. - Überraschenderweise eignet sich eine solche dsRNA zur Herstellung eines Medikaments zur Hemmung der Expression eines vorgegebenen Gens. Bei einer Verwendung von dsRNA wird die Hemmung im Vergleich zur Verwendung einzelsträngiger Oligoribonukleotide schon bei um eine Größenordnung geringeren Konzentrationen bewirkt. Die erfindungsgemäße Verwendung ermöglicht also die Herstellung besonders wirksamer Medikamente.

Nach weiterer Maßgabe der Erfindung wird die Aufgabe gelöst durch die Verwendung eines Vektors mit den Merkmalen des Anspruchs 114. - Die Verwendung eines solchen Vektors ermöglicht eine besonders wirksame Gentherapie.

Hinsichtlich vorteilhafter Ausgestaltungen des Medikaments und der Verwendung wird auf die Beschreibung der vorangegangenen Merkmale verwiesen.

Nachfolgend werden anhand der Figuren Ausführungsbeispiele der Erfindung näher erläutert. Es zeigen:
- Fig. 1: die schematische Darstellung eines Plasmids für die *in vitro*-Transkription mit T7- und SP6-Polymerase,
- Fig. 2: RNA nach Elektrophorese auf einem 8%igen Polyacrylamidgel und Ethidiumbromidfärbung,
- Fig. 3: eine Darstellung radioaktiver RNA-Transkripte nach Elektrophorese auf einem 8%igen Polyacrylamidgel mit 7 M Harnstoff mittels eines "Instant Imagers" und
- Fig. 4 a - e: Texas-Rot- und YFP-Fluoreszenz in murinen Fibroblasten.

### Ausführungsbeispiel 1:

Die Inhibition der Transkription wurde durch sequenzhomologe dsRNA in einem *in vitro*-Transkriptionssystem mit einem Kernextrakt aus humanen HeLa-Zellen nachgewiesen. Die DNA-Matrize für diesen Versuch war das mittels *Bam*HI linearisierte Plasmid pCMV1200.

### Herstellung der Matrizenplasmide:

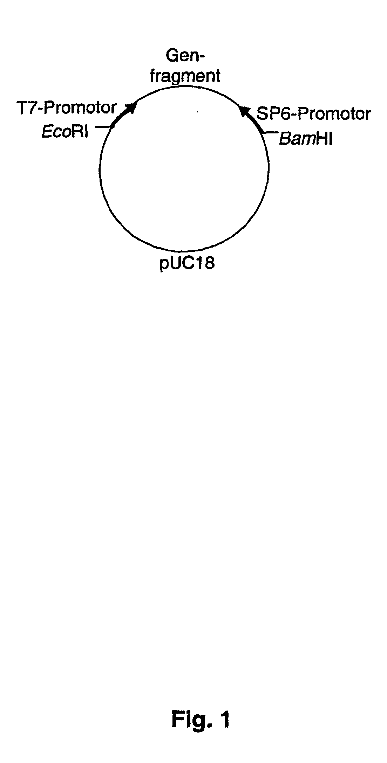

Zur Verwendung bei der enzymatischen Synthese der dsRNA wurde das in Fig. 1 dargestellte Plasmid konstruiert. Dazu wurde zunächst eine Polymerase-Kettenreaktion (PCR) mit der "positive control DNA" des HeLaScribe® Nuclear Extract *in vitro* Transkriptionskits der Firma Promega, Madison, USA als DNA-Matrize durchgeführt. Einer der verwendeten Primer enthielt die Sequenz einer *Eco*RI-Schnittstelle und des T7-RNA-Polymerase-Promotors gemäß Sequenzprotokoll Nr. 1. Der andere Primer enthielt die Sequenz einer *Bam*HI-Schnittstelle und des SP6-RNA-Polymerase-Promotors gemäß Sequenzprotokoll Nr. 2. Darüber hinaus wiesen beide Primer an ihren 3'-Enden identische bzw. komplementäre Bereiche zur DNA-Matrize auf. Die PCR wurde mittels des "*Taq PCR Core Kits"* der Firma Qiagen, Hilden, Deutschland nach Herstellerangaben durchgeführt. In einem Volumen von 100 µl wurden 1,5 mM MgCl₂, je 200 µM dNTP, je 0,5 µM Primer, 2,5 U *Taq*-DNA-Polymerase und etwa 100 ng "positive control DNA" als Matrize in PCR-Puffer eingesetzt. Nach der anfänglichen Denaturierung der Matrizen-DNA durch Erhitzen auf 94°C für 5 Minuten erfolgte die Amplifikation in 30 Zyklen von je 60 Sekunden Denaturierung bei 94°C, 60 Sekunden Annealing bei 5°C unterhalb der berechneten Schmelztemperatur der Primer und 1,5 - 2 Minuten Polymerisation bei 72°C. Nach einer Schlußpolymerisation von 5 Minuten bei 72°C wurden 5 µl des Reaktionsansatzes durch Agarosegelelektrophorese analysiert. Die Länge des so amplifizierten DNA-Fragmentes betrug 400 Basenpaare, wobei 340 Basenpaare der "positive control DNA" entsprachen. Das PCR-Produkt wurde aufgereinigt, mit *Eco*RI und *Bam*HI hydrolysiert und nach erneuter Aufreinigung zur Ligation mit einem ebenfalls durch *Eco*RI und *Bam*HI hydrolysierten pUC18 Vektor eingesetzt. Es erfolgte Transformation von *E. coli* XL1-blue. Das erhaltene Plasmid (pCMV5) trägt ein DNA-Fragment, das am 5'-Ende von dem T7- und am 3'-Ende von dem SP6-Promotor flankiert wird. Durch Linearisierung des Plasmids mit *Bam*HI kann es *in vitro* mit der T7-RNA-Polymerase zur *run-off*-Transkription einer 340 Nukleotide langen, in Sequenzprotokoll Nr. 3 dargestellten, einzelsträngigen RNA eingesetzt werden. Wird das Plasmid mit *Eco*RI linearisiert, kann es zur *run-off*-Transkription mit der SP6-RNA-Polymerase eingesetzt werden, wobei der komplementäre Strang entsteht. Entsprechend dem zuvor dargestellten Verfahren wurde auch eine 23 Nukleotide längere RNA synthetisiert. Dazu wurde eine in Sequenzprotokoll Nr. 4 dargestellte DNA über die *Eco*RI und *Bam*HI-Schnittstellen mit dem pUC18 Vektor ligiert.

Als DNA-Matrize für die in vitro-Transkription mit HeLa-Kernextrakt wurde das Plasmid pCMV1200 konstruiert. Dazu wurde ein 1191 bp großes EcoRI/BamHI-Fragment der im HeLaScribe® Nuclear Extract *in vitro* Transkriptionskit enthaltenen Positivkontroll-DNA mittels PCR amplifiziert. Das amplifizierte Fragment umfaßt den 828 bp großen "unmittelbar frühen" CMV-Promotor und ein 363 bp großes transkribierbares DNA-Fragment. Das PCR-Produkt wurde über "T-Überhang"-Ligation mit dem Vektor pGEM-T ligiert. Am 5'-Ende des Fragments ist eine BamHI-Schnittstelle. Das Plasmid wurde durch Hydrolyse mit BamHI linearisiert und als Matrize zur run-off-Transkription eingesetzt.

### In vitro-Transkription der komplementären Einzelstränge:

pCMV5-Plasmid-DNA wurde mit *Eco*RI bzw. *Bam*HI linearisiert. Sie wurde als DNA-Matrize für eine *in vitro*-Transkription der komplementären RNA-Einzelstränge mit SP6- bzw. T7-RNA-Polymerase verwendet. Dazu wurde das "Riboprobe *in vitro* Transcription" System der Firma Promega, Madison, USA eingesetzt. Nach Herstellerangaben wurden 2 µg linearisierte Plasmid-DNA in 100 µl Transkriptionspuffer und 40 U T7- oder SP6-RNA-Polymerase 5 - 6 Stunden bei 37°C inkubiert. Anschließend wurde die DNA-Matrize durch Zugabe von 2,5 µl RNase-freier DNase RQ1 und Inkubation für 30 Minuten bei 37°C abgebaut. Der Transkriptionsansatz wurde mit H₂O auf 300 µl aufgefüllt und durch Phenolextraktion gereinigt. Die RNA wurde durch Zugabe von 150 µl 7 M Ammoniumacatat und 1125 µl Ethanol gefällt und bis zur Hybridisierung bei -65°C aufbewahrt.

### Herstellung der RNA-Doppelstränge:

Zur Hybridisierung wurden 500 µl der in Ethanol aufbewahrten und gefällten einzelsträngigen RNA abzentrifugiert. Das resultierende Pellet wurde getrocknet und in 30 µl PIPES-Puffer, pH 6,4 in Gegenwart von 80 % Formamid, 400 mM NaCl und 1 mM EDTA aufgenommen. Jeweils 15 µl der komplementären Einzelstränge wurden zusammengegeben und für 10 Minuten auf 85°C erhitzt. Anschließend wurden die Ansätze bei 50°C über Nacht inkubiert und auf Raumtemperatur abgekühlt.

Bei der Hybridisierung wurden nur annähernd äquimolare Mengen der beiden Einzelstränge eingesetzt. Dadurch enthielten die dsRNA-Präparationen einzelsträngige RNA (ssRNA) als Kontamination. Um diese ssRNA-Kontaminationen zu entfernen, wurden die Ansätze nach der Hybridisierung mit den einzelstrangspezifischen Ribonukleasen RNase A aus Rinderpankreas und RNase T1 aus *Aspergillus oryzae* behandelt. RNase A ist eine für Pyrimidine spezifische Endoribonuklease. RNase T1 ist eine Endoribonuklease, die bevorzugt auf der 3'-Seite von Guanosinen schneidet. dsRNA ist kein Substrat für diese Ribonukleasen. Für die RNase-Behandlung wurde zu den Ansätzen in 300 µl Tris, pH 7,4, 300 mM NaCl und 5 mM EDTA 1,2 µl RNaseA in einer Konzentration von 10 mg/ml und 2 µl RNaseT1 in einer Konzentration von 290 µg/ml zugegeben. Die Ansätze wurden 1,5 Stunden bei 30°C inkubiert. Danach wurden die RNasen durch Zugabe von 5 µl Proteinase K in einer Konzentration von 20 mg/ml sowie 10 µl 20%iges SDS und Inkubation für 30 Minuten bei 37°C denaturiert. Die dsRNA wurde durch Phenol-Extraktion gereinigt und mit Ethanol gefällt. Um die Vollständigkeit des RNase-Verdaus überprüfen zu können, wurden zwei Kontrollansätze mit ssRNA analog zu den Hybridisierungsansätzen behandelt.

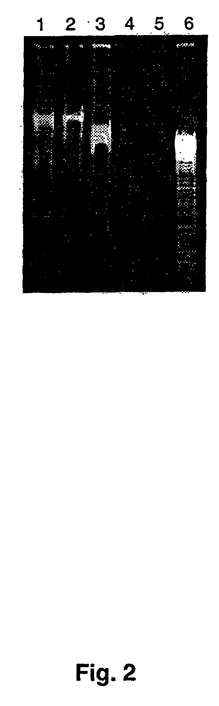

Das getrocknete Pellet wurde in 15 µl TE-Puffer, pH 6,5 aufgenommen und auf einem 8%igen Gel einer nativen Polyacrylamidgelelektrophorese unterzogen. Das Acrylamidgel wurde anschließend in einer Ethidiumbromidlösung gefärbt und in einem Wasserbad gespült. Fig. 2 zeigt die auf einem UV-Transilluminator sichtbar gemachte RNA. Die auf Spur 1 aufgetragene *sense-* und die auf Spur 2 aufgetragene *antisense*-RNA zeigten unter den gewählten Bedingungen ein anderes Laufverhalten als die auf Spur 3 aufgetragene dsRNA des Hybridisierungsansatzes. Die auf den Spuren 4 bzw. 5 aufgetragene RNase-behandelte *sense-* bzw. *antisense*-RNA erzeugte keine sichtbare Bande. Dies zeigt, daß die einzelsträngigen RNAs vollständig abgebaut wurden. Die auf Spur 6 aufgetragene RNase-behandelte dsRNA des Hybridisierungsansatzes ist resistent gegenüber der RNase-Behandlung. Die im nativen Gel im Vergleich zu der auf Spur 3 aufgetragenen dsRNA schneller wandernde Bande resultiert aus dsRNA, die frei von ssRNA ist. Neben der dominierenden Hauptbande treten nach der RNase-Behandlung schwächere, schneller wandernde Banden auf.

### In vitro-Transkriptions-Test mit menschlichem Zellkernextrakt:

Unter Verwendung des HeLaScribe® Nuclear Extract *in vitro* Transkriptionskits der Firma Promega, Madison, USA wurde die Transkriptionseffizienz des oben angegebenen, im Plasmid pCMV1200 enthaltenen, zur "positive control DNA" homologen DNA-Fragments in Gegenwart der sequenzhomologen dsRNA (dsRNA-CMV5) bestimmt. Außerdem wurde der Einfluß der nichtsequenzhomologen, dem "Gelb fluoreszierenden Protein" (YFP)-Gen entsprechenden dsRNA (dsRNA-YFP) untersucht. Diese dsRNA war analog zur seguenzhomologen dsRNA hergestellt worden. Die Sequenz eines Stranges dieser dsRNA ist Sequenzprotokoll Nr. 5 zu entnehmen. Als Matrize für die *run-off*-Transkription diente das Plasmid pCMV1200. Es trägt den "unmittelbar frühen" Promotor des Cytomegalievirus, der von der eukaryotischen RNA-Polymerase II erkannt wird, und ein transkribierbares DNA-Fragment. Die Transkription erfolgte mittels des HeLa-Kernextrakts, der alle notwendigen Proteine für eine Transkription enthält. Durch Zugabe von [•-³²P]rGTP zum Transkriptionsansatz wurde radioaktiv markiertes Transkript erhalten. Das verwendete [•-³²P]rGTP hatte eine spezifische Aktivität von 400 Ci/mmol, 10 mCi/ml. Pro Ansatz wurden 3 mM MgCl₂, je 400 µM rATP, rCTP, rUTP, 16 µM rGTP, 0,4 µM [•-³²P]rGTP und je nach Versuch 1 fmol linearisierte Plasmid-DNA und verschiedene Mengen an dsRNA in Transkriptionspuffer eingesetzt. Jeder Ansatz wurde mit H₂O auf ein Volumen von 8,5 µl aufgefüllt. Die Ansätze wurden vorsichtig gemischt. Zum Starten der Transkription wurden 4 U HeLa-Kernextrakt in einem Volumen von 4 µl zugegeben und für 60 Minuten bei 30°C inkubiert. Die Reaktion wurde durch Zugabe von 87,5 µl auf 30°C erwärmten Stopp-Mix beendet. Zur Entfernung der Proteine wurden die Ansätze mit 100 µl Phenol/Chloroform/Isoamylalkohol (25:24:1, v/v/v), gesättigt mit TE-Puffer, pH 5,0, versetzt und 1 Minute kräftig gemischt. Zur Phasentrennung wurde etwa 1 Minute bei 12000 rpm zentrifugiert und die obere Phase in ein neues Reaktionsgefäß überführt. Zu jedem Ansatz wurden 250 µl Ethanol zugegeben. Die Ansätze wurden gut gemischt und für mindestens 15 Minuten auf Trockeneis/Methanol inkubiert. Zur Präzipitation der RNA wurden die Ansätze 20 Minuten bei 12000 rpm und 4°C zentrifugiert. Der Überstand wurde verworfen. Das Pellet wurde 15 Minuten im Vakuum getrocknet und in 10 µl H₂O resüspendiert. Zu jedem Ansatz wurden 10 µl denaturierender Probenpuffer zugegeben. Die Trennung des freien GTP vom entstandenen Transkript erfolgte mittels denaturierender Polyacrylamid-Gelelektrophorese auf einem 8%igen Gel mit 7 M Harnstoff. Die bei der Transkription mit HeLa-Kernextrakt gebildeten RNA-Transkripte in denaturierendem Probenpuffer wurden für 10 Minuten auf 90°C erhitzt und 10 µl davon sofort in die frisch gespülten Probentaschen aufgetragen. Die Elektrophorese erfolgte bei 40 mA. Die Menge der bei der Transkription gebildeten radioaktiven ssRNA wurde nach der Elektrophorese mit Hilfe eines *Instant Imager* analysiert.

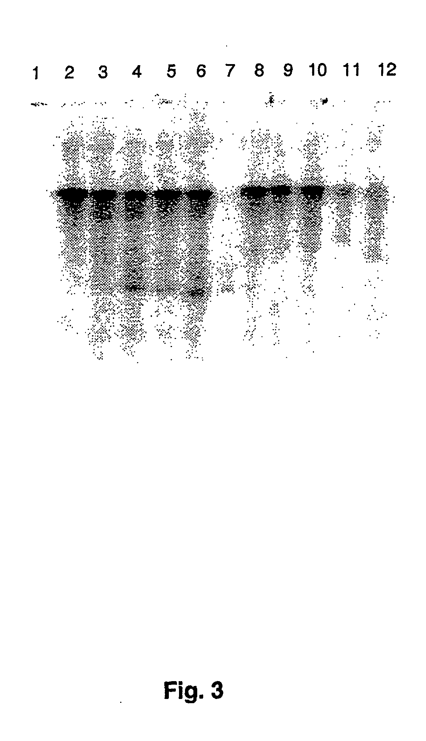

Fig. 3 zeigt die mittels des *Instant Imagers* dargestellte radioaktive RNA aus einem repräsentativen Tests. Es wurden aus folgenden Transkriptionsansätzen gewonne Proben aufgetragen:
- Spur 1:: ohne Matrizen-DNA, ohne dsRNA;
- Spur 2:: 50 ng Matrizen-DNA, ohne dsRNA;
- Spur 3:: 50 ng Matrizen-DNA, 0,5 µg dsRNA-YFP;
- Spur 4:: 50 ng Matrizen-DNA, 1,5 µg dsRNA-YFP;
- Spur 5:: 50 ng Matrizen-DNA, 3 µg dsRNA-YFP;
- Spur 6:: 50 ng Matrizen-DNA, 5 µg dsRNA-YFP;
- Spur 7:: ohne Matrizen-DNA, 1,5 dsRNA-YFP;
- Spur 8:: 50 ng Matrizen-DNA, ohne dsRNA;
- Spur 9:: 50 ng Matrizen-DNA, 0,5 µg dsRNA-CMV5;
- Spur 10:: 50 ng Matrizen-DNA, 1,5 µg dsRNA-CMV5;
- Spur 11:: 50 ng Matrizen-DNA, 3 µg dsRNA-CMV5;
- Spur 12:: 50 ng Matrizen-DNA, 5 µg dsRNA-CMV5;

Es zeigte sich eine deutliche Verringerung der Menge an Transkript in Gegenwart von sequenzhomologer dsRNA im Vergleich zum Kontrollansatz ohne dsRNA sowie auch zu den Ansätzen mit nicht-sequenzhomologer dsRNA-YFP. Die Positivkontrolle in Spur 2 zeigt, daß bei der *in vitro*-Transkription mit HeLa-Kernextrakt radioaktives Transkript gebildet wurde. Der Ansatz dient zum Vergleich mit den Transkriptionsansätzen, die in Gegenwart von dsRNA inkubiert worden waren. Die Spuren 3 bis 6 zeigen, daß die Zugabe von nicht-sequenzspezifischer dsRNA-YFP keinen Einfluß auf die Menge des gebildeten Transkripts hat. Die Spuren 9 bis 12 zeigen, daß die Zugabe einer zwischen 1,5 und 3 µg liegenden Menge sequenzspezifischer dsRNA-CMV5 zu einer Abnahme der gebildeten Transkript-Menge führt. Um auszuschließen, daß die beobachteten Effekte nicht auf der dsRNA, sondern auf einer möglicherweise bei der Herstellung der dsRNA unabsichtlich mitgeführten Kontamination beruhen, wurde eine weitere Kontrolle durchgeführt. Einzelstrang-RNA wurde wie oben beschrieben transkribiert und anschließend der RNase-Behandlung unterzogen. Mittels nativer Polyacrylamidgelelektrophorese konnte gezeigt werden, daß die ssRNA vollständig abgebaut worden war. Dieser Ansatz wurde wie die Hybridisierungsansätze einer Phenolextraktion und einer Ethanolfällung unterzogen und anschließend in TE-Puffer aufgenommen. Auf diese Weise wurde eine Probe erhalten, die keine RNA enthielt, aber mit den gleichen Enzymen und Puffern behandelt worden war wie die dsRNA. Spur 8 zeigt, daß der Zusatz dieser Probe keinen Einfluß auf die Transkription hatte. Die Abnahme des Transkripts bei Zugabe sequenzspezifischer dsRNA kann deshalb eindeutig der dsRNA selbst zugeschrieben werden. Die Reduzierung der Transkript-Menge eines Gens in Gegenwart von dsRNA bei einem menschlichen Transkriptionssystem zeigt eine Hemmung der Expression des entsprechenden Gens an. Dieser Effekt ist auf einen neuartigen, durch die dsRNA bedingten Mechanismus zurückzuführen.

### Ausführungsbeispiel 2:

Als Testsystem für diese *in vivo*-Experimente diente die murine Fibroblasten-Zellinie NIH3T3, ATCC CRL-1658. Mit Hilfe der Mikroinjektion wurde das YFP-Gen in die Zellkerne eingebracht. Die Expression des YFP wurde unter dem Einfluß gleichzeitig mittransfizierter sequenzhomologer dsRNA untersucht. Diese dsRNA-YFP ist über eine Länge von 315 bp zum 5'-Bereich des YFP-Gens homolog. Die Nukleotidsequenz eines Strangs der dsRNA-YFP ist in Sequenzprotokoll Nr. 5 wiedergegeben. Die Auswertung unter dem Fluoreszenzmikroskop erfolgte 3 Stunden nach Injektion anhand der grün-gelben Fluoreszenz des gebildeten YFP.

### Konstruktion des Matrizenplasmids und Herstellung der dsRNA:

Als Matrize für die Herstellung der YFP-dsRNA mittels T7- und SP6-*in vitro*-Transkription wurde ein Plasmid nach dem gleichen Prinzip wie im Ausführungsbeispiel 1 beschrieben konstruiert. Das gewünschte Genfragment wurde unter Verwendung des Primers *Eco*_T7_YFP gemäß Sequenzprotokoll Nr. 6 und *Bam*_SP6_YFP gemäß Sequenzprotokoll Nr. 7 mittels PCR amplifiziert und analog zu der obigen Beschreibung zur Herstellung der dsRNA verwendet. Die erhaltene dsRNA-YFP ist identisch mit der in Ausführungsbeispiel 1 als nicht-sequenzspezifische Kontrolle verwendeten dsRNA.

Es wurde eine am 3'-Ende der RNA gemäß Sequenzprotokoll Nr. 8 über eine C18-Linkergruppe chemisch mit dem 5'-Ende der komplementären RNA verknüpfte dsRNA (L-dsRNA) hergestellt. Dazu wurden mit Disulfid-Brücken modifizierte Synthone verwendet. Das 3'-terminale Synthon ist über den 3'-Kohlenstoff mit einer aliphatischen Linker-Gruppe über eine Disulfidbrücke an den festen Träger gebunden. Bei dem zum 3'-terminalen Synthon des einen Oligoribonukleotids komplementären 5'-terminalen Synthon des komplementären Oligoribonukleotids ist die 5'-Tritylschutzgruppe über einen weiteren aliphatischen Linker und eine Disulfidbrücke gebunden. Nach Synthese der beiden Einzelstränge, Entfernen der Schutzgruppen und Hybridisierung der komplementären Oligoribonukleotide gelangen die entstehenden Thiolgruppen in räumliche Nachbarschaft zueinander. Durch Oxidation werden die Einzelstränge über ihre aliphatischen Linker und eine Disulfidbrücke miteinander verknüpft. Anschließend erfolgt Reinigung mit Hilfe der HPLC.

### Vorbereitung der Zellkulturen:

Die Zellen wurden in DMEM mit 4,5 g/l Glucose, 10 % fötalem Rinderserum unter 7,5 % CO₂-Atmosphäre bei 37°C in Kulturschalen inkubiert und vor Erreichen der Konfluenz passagiert. Das Ablösen der Zellen erfolgte mit Trypsin/EDTA. Zur Vorbereitung der Mikroinjektion wurden die Zellen in Petrischalen überführt und bis zu Bildung von Mikrokolonien weiter inkubiert.

### Mikroinjektion:

Die Kulturschalen wurde zur Mikroinjektion für ca. 10 Minuten aus dem Inkubator genommen. Es wurde in ca. 50 Zellkerne pro Ansatz innerhalb eines markierten Bereichs unter Verwendung des Mikroinjektionssystems AIS der Firma Carl Zeiss, Göttingen, Deutschland einzeln injiziert. Anschließend wurden die Zellen weitere drei Stunden inkubiert. Für die Mikroinjektion wurden Borosilikat-Glaskapillaren der Firma Hilgenberg GmbH, Malsfeld, Deutschland mit einem Spitzendurchmesser unter 0,5 µm vorbereitet. Die Mikroinjektion wurde mit einem Mikromanipulator der Firma Narishige Scientific Instrument Lab., Tokyo, Japan durchgeführt. Die Injektionsdauer betrug 0,8 Sekunden, der Druck ca. 100 hPa. Für die Transfektion wurde das Plasmid pCDNA-YFP verwendet, das ein ca. 800 bp großes *Bam*HI/*Eco*RI-Fragment mit dem Gen des YFP im Vektor pcDNA3 enthält. Die in die Zellkerne injizierten Proben enthielten 0,01 µg/µl pCDNA-YFP sowie an Dextran-70000 gekoppeltes Texas-Rot in 14 mM NaCl, 3 mM KCl, 10 mM KPO₄, pH 7,5. Zusätzlich wurden ca. 100 pl RNA mit einer Konzentration von 1 µM, bzw. 375 µM im Fall der L-dsRNA, zugegeben.

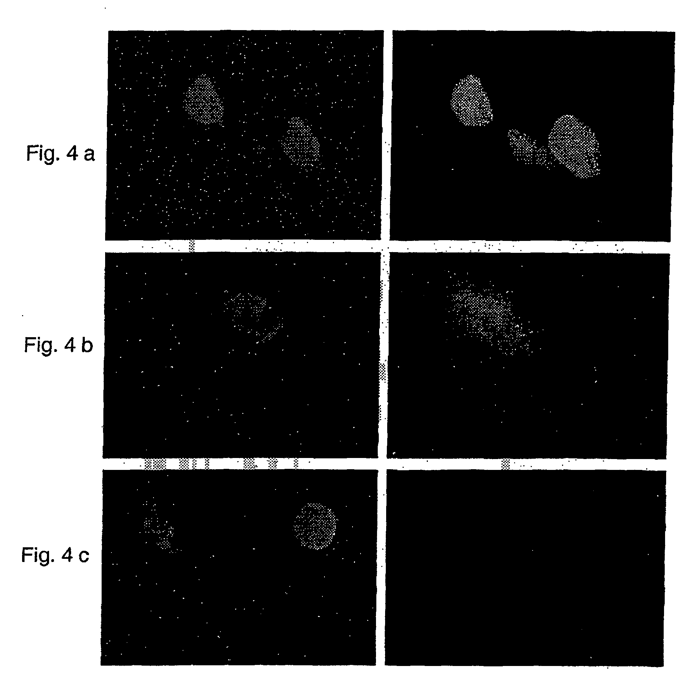

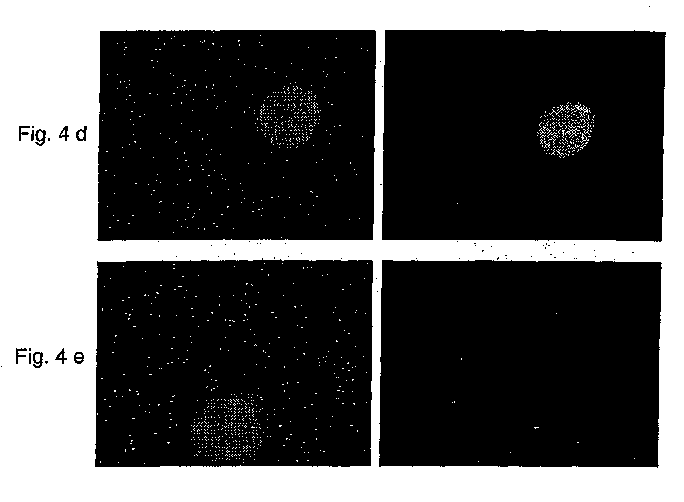

Die Zellen wurden bei Anregung mit Licht der Anregungswellenlänge von Texas-Rot, 568 nm, bzw. von YFP, 488 nm, mittels eines Fluoreszenzmikroskops untersucht. Einzelne Zellen wurden mittels einer digitalen Kamera dokumentiert. Die Figuren 4 a - e zeigen das Ergebnis für NIH3T3-Zellen. Bei den in Fig. 4 a gezeigten Zellen ist sense-YFP-ssRNA, in Fig. 4 b *antisense*-YFP-ssRNA, in Fig. 4 c dsRNA-YFP, in Fig. 4 d keine RNA und in Fig. 4 e L-dsRNA injiziert worden.

Das jeweils linke Feld zeigt die Fluoreszenz von Zellen, die mit 568 nm angeregt wurden. Rechts ist die Fluoreszenz derselben Zellen bei Anregung mit 488 nm zu sehen. Die Texas-Rot-Fluoreszenz aller dargestellten Zellen zeigt, daß die Injektionslösung erfolgreich in die Zellkerne appliziert wurde und getroffene Zellen nach drei Stunden noch lebendig waren. Abgestorbene Zellen zeigten keine Texas-Rot-Fluoreszenz mehr.

Die jeweils rechten Felder der Figuren 4 a und 4 b zeigen, daß die Expression des YFP bei Injektion der einzelsträngigen RNA in die Zellkerne nicht sichtbar inhibiert wurde. Das rechte Feld der Fig. 4 c zeigt Zellen, deren YFP-Fluoreszenz nach Injektion von dsRNA-YFP nicht mehr nachweisbar war. Fig. 4 d zeigt als Kontrolle Zellen, in die keine RNA injiziert worden war. Die in Fig. 4 e dargestellte Zelle zeigt durch die Injektion der L-dsRNA, die zum YFP-Gen sequenzhomologe Bereiche aufweist, eine nicht mehr nachweisbare YFP-Fluoreszenz. Dieses Ergebnis belegt, daß auch kürzere dsRNAs zur spezifischen Inhibition der Genexpression bei Säugern verwendet werden können, wenn die Doppelstränge durch chemische Verknüpfung der Einzelstränge stabilisiert werden.

### Literatur:

Asanuma, H., Ito, T., Yoshida, T., Liang, X. & Komiyama, M. (1999). Photoregulation der Bildung und Dissoziation eines DNA-Duplexes durch *cis*-*trans*-Isomerisierung einer Azobenzoleinheit. *Angew. Chem*. **111,** 2547-2549.
Azhayeva, E., Azhayev, A., Auriola, S., Tengvall, U., Urtti, A. & Lönnberg, H. (1997). Inhibitory properties of double helix forming circular oligonucleotides. *Nucl. Acids Res.* **25,** 4954-4961.
Castelli, J., Wood, K.A. & Youle, R.J. (1998). The 2-5A system in viral infection and apoptosis. *Biomed*. *Pharmacother.* **52,** 386-390.
Dolinnaya, N.G., Blumenfeld, M., Merenkova, I., Oretskaya, T.S., Krynetskaya, N.F., Ivanovskaya, M.G., Vasseur, M. & Shabarova, Z.A. (1993). Oligonucleotide circularization by template-directed chemical ligation. *Nucl*. *Acids Res.* **21,** 5403-5407.
Expert-Bezancon, A., Milet, M. & Carbon, P. (1983). Precise localization of several covalent RNA-RNA cross-link in *Escherichia coli* 16S RNA. *Eur*. *J*. *Biochem.* **136,** 267-274.
Fire, A., Xu, S., Montgomery, M.K., Kostas, S.A., Driver, S.E. & Mello, C.C. (1998). Potent and specific genetic interference by double-stranded RNA in *Caenorhabditis elegans. Nature* **391**, 806-811.
Gao, H., Yang, M., Patel, R. & Cook, A.F. (1995). Circulaization of oligonucleotides by disulfide bridge formation. *Nucl*. *Acids Res.* **23,** 2025-2029.
Gryaznov, S.M. & Letsinger, R.L. (1993). Template controlled coupling and recombination of oligonucleotide blocks containing thiophosphoryl groups. *Nucl*. *Acids Res.* **21,** 1403-1408.
Kaufman, R.J. (1999). Double-stranded RNA-activated protein kinase mediates virus-induced apoptosis: A new role for an old actor. *Proc*. *Natl*. *Acad*. *Sci*. *USA* **96,** 11693-11695.
Lipson, S.E. & Hearst, J.E. (1988). Psoralen cross-linking of ribosomal RNA. In *Methods in Enzymology* Anonymous pp. 330-341.
Liu, Z.R., Sargueil, B. & Smith, C.W. (1998). Detection of a novel ATP-dependent cross-linked protein at the 5' splice site-U1 small nuclear RNA duplex by methylene bluemediated photo-cross-linking. *Mol*. *Cell*. *Biol.* **18,** 6910-6920.
Micura, R. (1999). Cyclic oligoribonucleotides (RNA) by solidphase synthesis. *Chem*. *Eur*. *J*. **5,** 2077-2082.
Skripkin, E., Isel, C., Marquet, R., Ehresmann, B. & Ehresmann, C. (1996). Psoralen crosslinking between human immunodeficiency virus type 1 RNA and primer tRNA₃^{Lys}. *Nucl*. *Acids Res.* **24,** 509-514.
Wang, S. & Kool, E.T. (1994). Circular RNA oligonucleotides. Synthesis, nucleic acid binding properties, and a comparison with circular DNAs. *Nucl*. *Acids Res.* **22,** 2326-2333.
Wang, Z. & Rana, T.M. (1996). RNA conformation in the Tat-TAR complex determined by site-specific photo-cross-linking. *Biochem*. **35,** 6491-6499.
Watkins, K.P. & Agabian, N. (1991). *In vivo* UV cross-linking of U snRNAs that paticipate in trypanosome transsplicing. *Genes & Development* **5**, 1859-1869.
Wengel, J. (1999). Synthesis of 3'-*C*- and 4'-*C*-branched oligodeoxynucleotides and the development of locked nucleic acid (LNA). *Acc*. *Chem*. *Res.* **32,** 301-310.
Zwieb, C., Ross, A., Rinke, J., Meinke, M. & Brimacombe, R. (1978). Evidence for RNA-RNA cross-link formation in *Escherichia coli* ribosomes. *Nucl*. *Acids Res.* ***5,*** 2705-2720.

## Patentansprüche

1. Verfahren zur Hemmung der Expression eines vorgegebenen Zielgens in einer Zelle in vitro, wobei ein Oligoribonukleotid von aus zwei separaten RNA-Einzelsträngen gebildeter doppelsträngiger Struktur (dsRNA) in die Zelle eingeführt wird, wobei ein Strang der dsRMA einen zum Zielgen komplementären Bereich aufweist,
**dadurch gekennzeichnet, daß**
der komplementäre Bereich weniger als 25 aufeinanderfolgende Nukleotidpaare aufweist.

2. Verfahren nach Anspruch 1, wobei die dsRNA in micellare Strukturen, vorzugsweise in Liposomen, eingeschloasen wird.

3. Verfahren nach einem der vorhergehenden Ansprüche, wobei die dsRNA in virale natürliche Kapside oder in auf chemischem oder enzymatischem Weg hergestellte künstliche Kapside oder davon abgeleitete Strukturen eingeschlossen wird.

4. Verfahren nach einem der vorhergehenden Ansprüche, wobei das Zielgen in eukaryontischen Zellen exprimiert wird.

5. Verfahren nach einem der vorhergehenden Ansprüche, wobei das Zielgen aus der folgenden Gruppe ausgewählt ist: Onkogen, Cytokin-Gen, Id-Protein-Gen, Entwicklungsgen, Priongen.

6. Verfahren nach einem der vorhergehenden Ansprüche, wobei das zielgen in pathogenen Organismen, vorzugsweise in Plasmodien, exprimiert wird.

7. Verfahren nach einem der vorhergehenden Ansprüche, wobei das zielgen Bestandteil eines virus oder Viroids ist.

8. Verfahren nach Anspruch 7, wobei das Virus ein humanpathogenes Virus oder Viroid ist.

9. Verfahren nach Anspruch 7, wobei das Virus oder Viroid ein tier- oder pflanzenpathogenes Virus oder Viroid ist.

10. Verfahren nach einem der vorhergehenden Ansprüche, wobei die dsRNA abschnittsweise doppelsträngig ausgebildet ist.

11. Verfahren nach einem der vorhergehenden Ansprüche, wobei die Enden der dsRNA modifiziert werden, um einem Abbau in der Zelle oder einer Dissoziation in die Einzelstränge entgegenzuwirken.

12. Verfahren nach einem der vorhergehenden Ansprüche, wobei der durch die komplementären Nukleotidpaare bewirkte Zusammenhalt der doppelsträngigen Struktur durch mindestens eine, vorzugsweise zwei, weitere chemische Verknüpfung/en erhöht wird.

13. verfahren nach einem der vorhergehenden Ansprüche, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet wird.

14. Verfahren nach einem der vorhergehenden Ansprüche, wobei die chemische verknüpfung an mindestens einem, vorzugsweise an beiden, Enden der doppelsträngigen Struktur hergestellt wird.

15. Verfahren nach einem der vorhergehenden Ansprüche, wobei die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Polyethylenglycol-Ketten sind.

16. Verfahren nach einem der vorhergehenden Ansprüche, wobei die chemische Verknüpfung durch in der doppelsträngigen Struktur anstelle von Purinen benutzten Purinanaloga gebildet wird.

17. Verfahren nach einem der vorhergehenden Ansprüche, wobei die chemische Verknüpfung durch in die doppelsträngige Struktur eingeführte Azabenzoleinheiten gebildet wird.

18. Verfahren nach einem der vorhergehenden Ansprüche, wobei die chemische Verknüpfung durch in der doppelsträngigen Struktur anstelle von Nukleotiden benutzten verzweigten Nukleotidanaloga gebildet wird.

19. Verfahren nach einem der vorhergehenden Ansprüche, wobei zur Herstellung der chemischen Verknüpfung mindestenes eine der folgenden Gruppen benutzt wird. Methylenblau; bifunktionelle Gruppen, vorzugsweise Bis- (2-chlorethyl) -amin; N-acetyl-N'- (p-glyoxyl-benzoyl)-cystamin; 4-Thiouracil; Psoralen.

20. Verfahren nach einem der vorhergehenden Ansprüche, wobei. die chemische Verknüpfung durch an den Enden der doppelsträngigen Struktur angebrachte Thiophosphoryl-Gruppen gebildet wird.

21. Verfahren nach einem der vorhergehenden Ansprüche, wobei die chemische Verknüpfung an den Enden der doppelsträngigen Struktur durch Tripelhelix-Bindungen hergestellt wird.

22. Verfahren nach einem der vorhergehenden Ansprüche, wobei mindestens eine 2'-Hydroxylgruppe der Nukleotide der dsRNA in der doppelsträngigen Struktur durch eine chemische Gruppe, vorzugsweise eine 2'-Amino- oder eine 2'-Methylgruppe; ersetzt ist.

23. Verfahren nach einem der vorhergehenden Ansprüche, wobei mindestens ein Nukleotid in mindestens einem Strang der doppelsträngigen Struktur ein "locked nucleotide" mit einem, vorzugsweise durch eine 2'-O, 4'-C-Methylenbrücke, chemisch modifizierten Zuckerring ist.

24. Verfahren nach einem der vorhergehenden Ansprüche, wobei die dsRNA an mindestens ein von einem Virus stammendes, davon abgeleitetes oder ein synthetisch hergestelltes virales Hüllprotein gebunden, damit assoziiert oder davon umgeben wird.

25. Verfahren nach einem der vorhergehenden Ansprüche, wobei das Hüllprotein vom Polyomavirus abgeleitet ist.

26. Verfahren nach einem der vorhergehenden Ansprüche, wobei das Hüllprotein das Virus-Protein 1 (VP1) und/oder- das Virus-Protein 2 (VP2) des Polyomavirus enthält.

27. Verfahren nach einem der vorhergehenden Ansprüche, wobei bei Bildung eines Kapsids oder kapsidartigen Gebildes aus dem Hüllprotein die eine Seite zum Inneren des Kapsids oder kapsidartigen Gebildes gewandt ist.

28. Verfahren nach einem der vorhergehenden Ansprüche, wobei ein Strang der dsRNA zum primären oder prozessierten RNA-Transkript des Zielgens komplementär ist.

29. Verfahren nach einem der vorhergehenden Ansprüche, wobei die Zelle eine Vertebratenzelle oder eine menschliche Zelle ist.

30. Verfahren nach einem der vorhergehenden Ansprüche, wobei mindestens zwei voneinander verschiedene dsRNAs in die Zelle eingeführt werden, wobei ein Strang jeder dsRNA zumindest abschnittsweise komplementär zu jeweils einem von mindestens zwei verschiedenen Zielgenen ist.

31. Verfahren nach einem der vorhergehenden Ansprüche, wobei eines der Zielgene das PKR-Gen ist.

32. Medikament mit mindestens einem Oligoribonukleotid von doppelsträngiger aus zwei separaten RNA-Einzelsträngen gebildeter Struktur (dsRNA) zur Hemmung der Expression eines vorgegebenen Zielgens, wobei ein Strang der dsRNA einen zum Zielgen komplementären Bereich aufweist,
**dadurch gekennzeichnet, daß**
der komplementäre Bereich weniger als 25 aufeinanderfolgende Nukleotidpaare aufweist.

33. Medikament nach Anspruch 32, wobei die dsRNA verpackt in micellare Strukturen, vorzugsweise in Liposomen, vorliegt.

34. Medikament nach einem der Ansprüche 32 oder 33, wobei die dsRNA in virale natürliche Kapside oder in auf chemischem oder enzymatischem Weg hergestellte künstliche Kapside oder davon abgeleitete Strukturen eingeschlossen ist.

35. Medikament nach einem der Ansprüche 32 bis 34, wobei das Zielgen in eukaryontischen Zellen exprimierbar ist.

36. Medikament nach einem der Ansprüche 32 bis 35, wobei das Zielgen aus der folgenden Gruppe ausgewählt ist: Onkogen, cytokin-Gen, Id-Protein-Gen, Entwicklungsgen, Priongen.

37. Medikament nach einem der Ansprüche 32 bis 36, wobei das Zielgen in pathogenen Organismen, vorzugsweise in Plasmodien, exprimierbar ist.

38. Medikament nach einem der Ansprüche 32 bis 37, wobei das zielgen Bestandteil eines Virus oder Viroids ist.

39. Medikament nach Anspruch 38, wobei das Virus ein humanpathogenes Virus oder Viroid ist.

40. Medikament nach Anspruch 38, wobei das Virus oder Viroid ein tierpathogenes Virus oder Viroid ist.

41. Medikament nach einem der Ansprüche 32 bis 40, wobei die dsRNA abschnittsweise doppelsträngig ausgebildet ist.

42. Medikament nach einem der Ansprüche 32 bis 40, wobei die Enden der dsRNA modifiziert werden, um einem Abbau in der Zelle oder einer Dissoziation in die Einzelstränge entgegenzuwirken.

43. Medikament nach einem der Ansprüche 32 bis 42, wobei der durch die komplementären Nukleotidpaare bewirkte Zusammenhalt der doppelsträngigen Struktur durch mindestens eine, vorzugsweise zwei, weitere chemische Verknüpfung/en erhöht wird.

44. Medikament nach einem der Ansprüche 32 bis 43, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet wird.

45. Medikament nach einem der Ansprüche 32 bis 44, wobei die chemische Verknüpfung an mindestens einem, vorzugsweise an beiden, Enden der doppelsträngigen Struktur hergestellt wird.

46. Medikament nach einem der Ansprüche 32 bis 45, wobei die chemische Verknüpfung mittels einer oder mehrerer Verbindungsgruppen gebildet ist, wobei die Verbindungsgruppen vorzugsweise Poly-(oxyphosphinicooxy-1,3-propandiol)- und/oder Polyethylenglycol-Ketten sind.

47. Medikament nach einem der Ansprüche 32 bis 46, wobei die chemische Verknüpfung durch in der doppelsträngigen Struktur anstelle von Purinen benutzten Purinanaloga gebildet ist.

48. Medikament nach einem der Ansprüche 32 bis 47, wobei die chemische Verknüpfung durch in die doppelsträngige Struktur eingeschaltete Azabenzoleinheiten gebildet ist.

49. Medikament nach einem der Ansprüche 32 bis 48, wobei die chemische Verknüpfung durch in der doppelsträngigen Struktur anstelle von Nukleotiden benutzten verzweigten Nukleotidanaloga gebildet ist.

50. Medikament nach einem der Ansprüche 32 bis 49, wobei zur Herstellung der chemischen Verknüpfung mindestenes eine der folgenden Gruppen benutzt wird: Methylenblau; bifunktionelle Gruppen, vorzugsweise Bis-(2-chlorethyl)-amin; N-acetyl-N⁻-(pglyoxyl-benzoyl)-cystamin; 4-Thiouracil; Psoralen.

51. Medikament nach einem der Ansprüche 32 bis 50, wobei die chemische Verknüpfung durch an den Enden der doppelsträngigen Struktur vorgesehene Thiophosphoryl-Gruppen gebildet ist.

52. Medikament nach einem der Ansprüche 32 bis 51, wobei die chemische Verknüpfung an den Enden der doppelsträngigen Struktur vorgesehene Tripelhelix-Bindungen sind.

53. Medikament nach einem der Ansprüche 32 bis 52, wobei mindestens eine 2'-Hydroxylgruppe der Nukleotide der dsRNA in der doppelsträngigen Struktur durch eine chemische Gruppe, vorzugsweise eine 2'-Amino- oder eine 2'-Methylgruppe, ersetzt ist.

54. Medikament nach einem der Ansprüche 32 bis 53, wobei mindestens ein Nukleotid in mindestens einem Strang der doppelsträngigen struktur ein "locked nucleotide" mit einem, vorzugsweise durch eine 2'-O, 4'-C-Methylenbrücke, chemisch modifizierten Zuckerring ist.

55. Medikament nach einem der Ansprüche 32 bis 54, wobei die dsRNA an mindestens ein von einem Virus stammendes, davon abgeleitetes oder ein synthetisch hergestelltes virales Hüllprotein gebunden, damit assoziiert oder davon umgeben ist.

56. Medikament nach einem der Ansprüche 32 bis 55, wobei das Hüllprotein vom Polyomavirus abgeleitet ist.

57. Medikament nach einem der Ansprüche 32 bis 56, wobei das Hüllprotein das Virus-Protein 1 (VP1) und/oder das Virus-Protein 2 (VP2) des Polyomavirus enthält.

58. Medikament nach einem der Ansprüche 32 bis 57, wobei bei Bildung eines Kapsids oder kapsidartigen Gebildes aus dem Hüllprotein die eine Seite zum Inneren des Kapsids oder kapsidartigen Gebildes gewandt ist.

59. Medikament nach einem der Ansprüche 32 bis 58, wobei ein Strang der dsRMA zum primären oder prozessierten RNA-Transkript des Zielgens komplementär ist.

60. Medikament nach einem der Ansprüche 32 bis 59, wobei die Zelle eine Vertebratenzelle oder eine menschliche Zelle ist.

61. Medikament nach einem der Ansprüche 32 bis 60, wobei darin mindestens zwei voneinander verschiedene dsRNAs enthalten sind, wobei ein Strang jeder dsRMA zumindest abschnittsweise komplementär zu jeweils einem von mindestens zwei verschiedenen Zielgenen ist.

62. Medikament nach Anspruch 61, wobei eines der Zielgene das PKR-Gen ist.

63. Wirkstoff mit mindestens einem Oligoribonukleotid von doppelsträngiger aus zwei separaten RNA-Einzelsträngen gebildeter Struktur (dsRNA) zur Hemmung der Expression eines vorgegebenen Zielgens, wobei ein Strang der dsRNA einen zum zielgen komplementären Bereich aufweist, und wobei das Zielgen Bestandteil eines pflanzenpathogenen Virus oder Viroids ist,
**dadurch gekennzeichnet, daß**
der komplementäre Bereich weniger als 25 aufeinanderfolgende Nukleotidpaare aufweist.

64. Wirkstoff nach Anspruch 63, wobei das Zielgen in eukaryontischen Zellen exprimierbar ist.

65. Wirkstoff nach Anspruch 63 oder 64, wobei die dsRNA abschnittsweise doppelsträngig ausgebildet ist.

66. Wirkstoff nach einem der Ansprüche 63 bis 65, wobei die Enden der dsRMA modifiziert worden, um einem Abbau in der Zelle oder einer Dissoziation in die Einzelstränge entgegenzuwirken.

67. Wirkstoff nach einem der Ansprüche 63 bis 66, wobei der durch die komplementären Nukleotidpaare bewirkte Zusammenhalt der doppelsträngigen Struktur durch mindestens eine, vorzugsweise zwei, weitere chemische Verknüpfung/en erhöht wird.

68. Wirkstoff nach einem der Ansprüche 63 bis 67, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet wird.

69. Wirkstoff nach einem der Ansprüche 63 bis 69, wobei die chemische Verknüpfung an mindestens einem, vorzugsweise an beiden, Enden der doppelsträngigen Struktur hergestellt wird.

70. Wirkstoff nach einem der Ansprüche 63 bis 69, wobei die chemische Verknüpfung mittels einer oder mehrerer Verbindungsgruppen gebildet ist, wobei die Verbindungsgruppen vorzugsweise Poly-(oxyphosphinicooxy-1,3-propandiol)-und/oder Polyethylenglycol-Ketten sind.

71. Wirkstoff nach einem der Ansprüche 63 bis 70, wobei, die chemische Verknüpfung durch in der doppelsträngigen Struktur anstelle von Purinen benutzten Purinanaloga gebildet ist.

72. Wirkstoff nach einem der Ansprüche 63 bis 71, wobei die chemische Verknüpfung durch in die doppelsträngige Struktur eingeschaltete Azabenzoleinheiten gebildet ist.

73. Wirkstoff nach einem der Ansprüche 63 bis 72, wobei die chemische Verknüpfung durch in der doppelsträngigen Struktur anstelle von Nukleotiden benutzten verzweigten Nukleotidanaloga gebildet ist.

74. Wirkstoff nach einem der Ansprüche 63 bis 73, wobei zur Herstellung der chemischen Verknüpfung mindestenes eine der folgenden Gruppen benutzt wird: Methylenblau; bifunktionelle Gruppen, vorzugsweise Bis-(2-chlorethyl)-amin; N-acetyl-N'-(pglyoxyl-benzoyl)-cystamin; 4-Thiouracil; Psoralen.

75. Wirkstoff nach einem der Ansprüche 63 bis 74, wobei die chemische Verknüpfung durch an den Enden der doppelsträngigen Struktur vorgesehene Thiophosphoryl-Gruppen gebildet ist.

76. Wirkstoff nach einem der Ansprüche 63 bis 75, wobei die chemische Verknüpfung an den Enden der doppelsträngigen Struktur vorgesehene Tripelhelix-Bindungen sind.

77. Wirkstoff nach einem der Ansprüche 63 bis 76, wobei mindestens eine 2'-Hydroxylgruppe der Nukleotide der dsRNA in der doppelsträngigen Struktur durch eine chemische Gruppe, vorzugsweise eine 2'-Amino- oder eine 2'-Methylgruppe, ersetzt ist.

78. Wirkstoff nach einem der Ansprüche 63 bis 77, wobei mindestens ein Nukleotid in mindestens einem Strang der doppelsträngigen Struktur ein "locked nucleotide" mit einem, vor-. zugsweise durch eine 2'-O, 4'-C-Methylenbrücke, chemisch modifizierten Zuckerring ist.

79. Wirkstoff nach einem der Ansprüche 63 bis 78, wobei ein Strang der dsRNA zum primären oder prozessierten RNA-Transkript des Zielgens komplementär ist.

80. Wirkstoff nach einem der Ansprüche 63 bis 79, wobei darin mindestens zwei voneinander verschiedene dsRNAs enthalten sind, wobei ein Strang jeder dsRNA zumindest abschnittsweise komplementär zu jeweils einem von mindestens zwei verschiedenen Zielgenen ist.

81. Verwendung eines Oligoribonukleotids von doppelsträngiger aus zwei separaten RNA-Einzelsträngen gebildeter Struktur-(dsRNA) zur Herstellung eines Medikaments oder Wirkstoffs zur Hemmung der Expression eines vorgegebenen Zielgens, wobei ein Strang der dsRNA einen zum Zielgen komplementären Bereich aufweist,
**dadurch gekennzeichnet, daß**
der komplementäre Bereich weniger als 25 aufeinanderfolgende Nukleotidpaare aufweist.

82. Verwendung nach Anspruch 81, wobei die dsRMA verpackt in micellare Strukturen, vorzugsweise in Liposomen, vorliegt.

83. Verwendung nach einem der Ansprüche 81 oder 82, wobei die dsRNA in virale natürliche Kapside oder in auf chemischem oder enzymatischem Weg hergestellte künstliche Kapside oder davon abgeleitete Strukturen eingeschlossen ist.

84. Verwendung nach einem der Ansprüche 81 bis 83, wobei das Zielgen in eukaryontischen Zellen exprimierbar ist.

85. Verwendung nach einem der Ansprüche 81 bis 84, wobei das Zielgen aus der folgenden Gruppe ausgewählt ist: Onkogen, Cytokin-Gen, Id-Protein-Gen, Entwicklungsgen, Priongen.

86. Verwendung nach einem der Ansprüche 81 bis 85, wobei das Zielgen in pathogenen Organismen, vorzugsweise in Plasmodien, exprimierbar ist.

87. Verwendung nach einem der Ansprüche 81 bis 86, wobei das Zielgen Bestandteil eines Virus oder Viroids ist.

88. Verwendung nach Anspruch 87, wobei das Virus ein humanpathogenes Virus oder Viroid ist.

89. Verwendung nach Anspruch 87, wobei das Virus oder Viroid ein tier- oder pflanzenpathogenes Virus oder Viroid ist.

90. Verwendung nach einem der Ansprüche 81 bis 89, wobei die dsRNA abschnittsweise doppelsträngig ausgebildet ist.

91. Verwendung nach einem der Ansprüche 81 bis 90, wobei die Enden der dsRNA modifiziert sind, um einem Abbau in der Zelle oder einer Dissoziation in die Einzelgtränge entgegenzuwirken.

92. Verwendung nach einem der Ansprüche 81 bis 91, wobei der durch die Nukleotidpaare bewirkte Zusammenhalt der doppelsträngigen Struktur durch mindestens eine, vorzugsweise zwei, weitere chemische Verknüpfung/en erhöht ist.

93. Verwendung nach einem der Ansprüche 81 bis 92, wobei die chemische Verknüpfung durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenko-ordination gebildet ist.

94. Verwendung nach einem der Ansprüche 81 bis 93, wobei die chemische Verknüpfung an mindestens einem, vorzugsweise an beiden, Enden der doppelsträngigen Struktur hergestellt ist.

95. Verwendung nach einem der Ansprüche 81 bis 94, wobei die chemische Verknüpfung mittels einer oder mehrerer Verbindungsgruppen gebildet ist, wobei die Verbindungsgruppen vorzugsweise Poly-(oxyphosphinicooxy-1,3-propandiol)- und/oder Polyethylenglycol-Ketten sind.

96. Verwendung nach einem der Ansprüche 81 bis 95, wobei die chemische Verknüpfung durch in der doppelsträngigen Struktur anstelle von Purinen benutzten Purinanaloga gebildet ist.

97. Verwendung nach einem der Ansprüche 81 bis 96, wobei die chemische Verknüpfung durch in die doppelsträngigen Struktur eingeführte Azabenzoleinheiten gebildet ist.

98. Verwendung nach einem der Ansprüche 81 bis 97, wobei die chemische Verknüpfung durch in der doppelsträngigen Struktur anstelle von Nukleotiden benutzten verzweigten Nukleotidanaloga gebildet ist.

99. Verwendung nach einem der Ansprüche 81 bis 98, wobei zur Herstellung der chemischen Verknüpfung mindestenes eine der folgenden Gruppen benutzt wird: Methylenblau; bifunktionelle Gruppen, vorzugsweise Bis- (2-chlorethyl) -amin; N-acetyl-N'- (p-glyoxyl-benzoyl)-cystamin; 4-Thiouracil; Psoralen.

100. Verwendung nach einem der Ansprüche 81 bis 99, wobei die chemische Verknüpfung durch an den Enden der doppelsträngigen Struktur angebrachte Thiophosphoryl-Gruppen gebildet ist.

101. Verwendung nach einem der Ansprüche 81 bis 100, wobei die chemische Verknüpfung an den Enden der doppelsträngigen Struktur durch Tripelhelix-Bindungen hergestellt ist.

102. Verwendung nach einem der Ansprüche 81 bis 101, wobei mindestens eine 2'-Hydroxylgruppe der Nukleotide der dsRNA in der doppelsträngigen Struktur durch eine chemische Gruppe, vorzugsweise eine 2'-Amino- oder eine 2'-Methylgruppe, ersetzt ist.

103. Verwendung nach einem der Ansprüche 81 bis 102, wobei mindestens ein Nukleotid in mindestens einem Strang der doppelsträngigen Struktur ein "locked nucleotide" mit einem, vorzugsweise durch eine 2'-O, 4'-C-Methylenbrücke, chemisch modifizierten Zuckerring ist.

104. Verwendung nach einem der Ansprüche 81 bis 103, wobei die dsRNA an mindestens ein von einem Virus stammendes, davon abgeleitetes oder ein synthetisch hergestelltes virales Hüllprotein gebunden, damit assoziiert oder davon umgeben ist.

105. Verwendung nach einem der Ansprüche 81 bis 104, wobei das Hüllprotein vom Polyomavirus abgeleitet ist.

106. Verwendung nach einem der Ansprüche 81 bis 105, wobei das Hüllprotein das Virus-Protein 1 (VP1) und/oder das Virus-Protein 2 (VP2) des Polyomavirus enthält.

107. Verwendung nach einem der Ansprüche 81 bis 106, wobei bei Bildung eines Kapsids oder kapsidartigen Gebildes aus dem Hüllprotein die eine Seite zum Inneren des Kapsids oder kapsidartigen Gebildes gewandt ist.

108. Verwendung nach einem der Ansprüche 81 bis 107, wobei ein Strang der dsRNA zum primären oder prozessierten RNA-Transkript des Zielgens komplementär ist.

109. Verwendung nach einem der Ansprüche 81 bis 108, wobei die Zelle eine Vertebratenzelle oder eine menschliche Zelle ist.

110. Verwendung nach einem der Ansprüche 81 bis 109, wobei mindestens zwei voneinander verschiedene dsRNAs verwendet werden, wobei ein Strang jeder dsRNA zumindest abschnittsweise komplementär zu jeweils einem von mindestens zwei verachiedenen Zielgenen ist.

111. Verwendung nach Anspruch 110, wobei eines der Zielgene das PKR-Gen ist.

112. Verwendung nach einem der Ansprüche 81 bis 111, wobei das Medikament in die Blutbahn oder das Interstitium des zu therapierenden Organismus injizierbar ist.

113. Verwendung nach einem der Ansprüche 81 bis 112, wobei die dsRNA in Bakterien oder Mikroorganismen aufgenommen ist.

114. Verwendung eines Vektors zur Kodierung mindestens eines Oligoribonukleotids von doppelsträngiger aus zwei separaten RNA-Einzelsträngen gebildeter Struktur (dsRNA) zur Herstellung eines Medikaments oder Wirkstoffs zur Hemmung der Expression eines vorgegebenen Zielgens, wobei ein Strang der dsRNA einen zum Zielgen komplementären Bereich aufweist,
**dadurch gekennzeichnet, daß**
der komplementäre Bereich weniger als 25 aufeinanderfolgende Nukleotidpaare aufweist.

115. Verwendung nach Anspruch 114, wobei das Zielgen in eukaryontischen Zellen exprimierbar ist.

116. Verwendung nach Anspruch 114 oder 115, wobei das Zielgen aus der folgenden Gruppe ausgewählt ist: Onkogen, Cytokin-Gen, Id-Protein-Gen, Entwicklungsgen, Priongen.

117. Verwendung nach einem der Ansprüche 114 bis 116, wobei das Zielgen in pathogenen Organismen, vorzugsweise in Plasmodien, exprimierbar ist.

118. Verwendung nach einem der Ansprüche 114 bis 117, wobei das Zielgen Bestandteil eines Virus oder Viroids ist.

119. Verwendung nach Anspruch 118, wobei das Virus ein humanpathogenes Virus oder Viroid ist.

120. Verwendung nach Anspruch 118, wobei das Virus oder Viroid ein tier- oder pflanzenpathogenes Virus oder Viroid ist.

121. Verwendung nach einem der Ansprüche 114 bis 120, wobei die dsRNA abschnittsweise doppelsträngig ausgebildet ist.

122. Verwendung nach einem der Ansprüche 114 bis 121, wobei ein Strang der dsRNA zum primären oder prozessierten RNA-Transkript des Zielgens komplementär ist.

123. Verwendung nach einem der Ansprüche 114 bis 122, wobei die Zelle eine Vertebratenzelle oder eine menschliche Zelle ist.

124. Verwendung nach einem der Ansprüche 114 bis 123, wobei mindestens zwei voneinander verschiedene dsRNAs verwendet werden, wobei ein Strang jeder dsRNA zumindest abschnittsweise komplementär zu jeweils einem von mindestens zwei verschiedenen Zielgenen ist.

125. Verwendung nach Anspruch 124, wobei eines der Zielgene das PKR-Gen ist.

## Claims

1. Method for inhibiting the expression of a given target gene in a cell in vitro, where an oligoribonucleotide with double-stranded structure (dsRNA) formed by two separate RNA single strands is introduced into the cell, where one strand of the dsRNA has a region which is complementary to the target gene,
**characterized in that**
the complementary region has less than 25 successive nucleotide pairs.

2. Method according to claim 1, where the dsRNA is enclosed by micellar structures, preferably by liposomes.

3. Method according to either of the preceding claims, where the dsRNA is enclosed by natural viral capsids or by chemically or enzymatically produced artificial capsids or structures derived therefrom.

4. Method according to one of the preceding claims, where the target gene is expressed in eukaryotic cells.

5. Method according to one of the preceding claims, where the target gene is selected from the following group: oncogene, cytokin gene, Id-protein gene, development gene, prion gene.

6. Method according to one of the preceding claims, where the target gene is expressed in pathogenic organisms, preferably in plasmodia.

7. Method according to one of the preceding claims, where the target gene is part of a virus or viroid.

8. Method according to claim 7, where the virus is a virus or viroid which is pathogenic for humans.

9. Method according to claim 7, where the virus or viroid is a virus or viroid which is pathogenic for animals or phytopathogenic.

10. Method according to one of the preceding claims, where segments of the dsRNA are in double-stranded form.

11. Method according to one of the preceding claims, where the ends of the dsRNA are modified in order to counteract degradation in the cell or dissociation into the single strands.

12. Method according to one of the preceding claims, where the cohesion of the double-stranded structure, which is caused by the complementary nucleotide pairs, is increased by at least one, preferably two, further chemical linkage(s).

13. Method according to one of the preceding claims, where the chemical linkage is formed by a covalent or ionic bond, a hydrogen bond, hydrophobic interactions, preferably van-der-Waals or stacking interactions, or by metal-ion coordination.

14. Method according to one of the preceding claims, where the chemical linkage is generated at at least one, preferably both, ends of the double-stranded structure.

15. Method according to one of the preceding claims, where the chemical linkage is formed by means of one or more compound groups, the compound groups preferably being poly(oxyphosphinicooxy-1,3-propanediol) and/or polyethylene glycol chains.

16. Method according to one of the preceding claims, where the chemical linkage is formed by purine analogs used in the double-stranded structure in place of purines.

17. Method according to one of the preceding claims, where the chemical linkage is formed by azabenzene units introduced into the double-stranded structure.

18. Method according to one of the preceding claims, where the chemical linkage is formed by branched nucleotide analogs used in the double-stranded structure in place of nucleotides.

19. Method according to one of the preceding claims, where at least one of the following groups is used for generating the chemical linkage: methylene blue; bifunctional groups, preferably bis(2-chloroethyl)amine; N-acetyl-N'-(p-glyoxylbenzoyl)cystamine; 4-thiouracil; psoralene.

20. Method according to one of the preceding claims, where the chemical linkage is formed by thiophosphoryl groups provided at the ends of the double-stranded structure.

21. Method according to one of the preceding claims, where the chemical linkage at the ends of the double-stranded structure is formed by triple-helix bonds.

22. Method according to one of the preceding claims, where at least one 2'-hydroxyl group of the nucleotides of the dsRNA in the double-stranded structure is replaced by a chemical group, preferably a 2'-amino or a 2'-methyl group.

23. Method according to one of the preceding claims, where at least one nucleotide in at least one strand of the double-stranded structure is a locked nucleotide with a sugar ring which is chemically modified, preferably by a 2'-O, 4'-C-methylene bridge.

24. Method according to one of the preceding claims, where the dsRNA is bound to, associated with or surrounded by, at least one viral coat protein which originates from a virus, is derived therefrom or has been prepared synthetically.

25. Method according to one of the preceding claims, where the coat protein is derived from polyomavirus.

26. Method according to one of the preceding claims, where the coat protein contains the polyomavirus virus protein 1 (VP1) and/or virus protein 2 (VP2).

27. Method according to one of the preceding claims, where, when a capsid or capsid-type structure is formed from the coat protein, one side faces the interior of the capsid or capsid-type structure.

28. Method according to one of the preceding claims, where one strand of the dsRNA is complementary to the primary or processed RNA transcript of the target gene.

29. Method according to one of the preceding claims, where the cell is a vertebrate cell or a human cell.

30. Method according to one of the preceding claims, where at least two dsRNAs which differ from each other are introduced into the cell, where at least segments of one strand of each dsRNA are complementary to in each case one of at least two different target genes.

31. Method according to one of the preceding claims, where one of the target genes is the PKR gene.

32. Medicament with at least one oligoribonucleotide with double-stranded structure (dsRNA) formed by two separate RNA single strands for inhibiting the expression of a given target gene, where one strand of the dsRNA has a region which is complementary to the target gene,
**characterized in that**
the complementary region has less than 25 successive nucleotide pairs.

33. Medicament according to claim 32, where the dsRNA is enclosed by micellar structures, preferably by liposomes.

34. Medicament according to either of claims 32 or 33, where the dsRNA is enclosed by natural viral capsids or by chemically or enzymatically produced artificial capsids or structures derived therefrom.

35. Medicament according to one. of claims 32 to 34, where the target gene can be expressed in eukaryotic cells.

36. Medicament according to one of claims 32 to 35, where the target gene is selected from the following group: oncogene, cytokin gene, Id-protein gene, development gene, prion gene.

37. Medicament according to one of claims 32 to 36, where the target gene can be expressed in pathogenic organisms, preferably in plasmodia.

38. Medicament according to one of claims 32 to 37, where the target gene is part of a virus or viroid.

39. Medicament according to claim 38, where the virus is a virus or viroid which is pathogenic for humans.

40. Medicament according to claim 38, where the virus or viroid is a virus or viroid which is pathogenic for animals.

41. Medicament according to one of claims 32 to 40, where segments of the dsRNA are in double-stranded form.

42. Medicament according to one of claims 32 to 40, where the ends of the dsRNA are modified in order to counteract degradation in the cell or dissociation into the single strands.

43. Medicament according to one of claims 32 to 42, where the cohesion of the double-stranded structure, which is caused by the complementary nucleotide pairs, is increased by at least one, preferably two, further chemical linkage(s).

44. Medicament according to one of claims 32 to 43, where the chemical linkage is formed by a covalent or ionic bond, a hydrogen bond, hydrophobic interactions, preferably van-der-Waals or stacking interactions, or by metal-ion coordination.

45. Medicament according to one of claims 32 to 44, where the chemical linkage is generated at at least one, preferably both, ends of the double-stranded structure.

46. Medicament according to one of claims 32 to 45, where the chemical linkage is formed by means of one or more compound groups, the compound groups preferably being poly(oxyphosphinicooxy-1,3-propanediol) and/or polyethylene glycol chains.

47. Medicament according to one of claims 32 to 46, where the chemical linkage is formed by purine analogs used in the double-stranded structure in place of purines.

48. Medicament according to one of claims 32 to 47, where the chemical linkage is formed by azabenzene units inserted into the double-stranded structure.

49. Medicament according to one of claims 32 to 48, where the chemical linkage is formed by branched nucleotide analogs used in the double-stranded structure in place of nucleotides.

50. Medicament according to one of claims 32 to 49, where at least one of the following groups is used for generating the chemical linkage: methylene blue; bifunctional groups, preferably bis(2-chloroethyl)amine; N-acetyl-N'-(p-glyoxylbenzoyl) - cystamine; 4-thiouracil; psoralene.

51. Medicament according to one of claims 32 to 50, where the chemical linkage is formed by thiophosphoryl groups provided at the ends of the double-stranded structure.

52. Medicament according to one of claims 32 to 51, where the chemical linkage are [sic] triple-helix bonds provided at the ends of the double-stranded structure.

53. Medicament according to one of claims 32 to 52, where at least one 2'-hydroxyl group of .the nucleotides of the dsRNA in the double-stranded structure is replaced by a chemical group, preferably a 2'-amino or a 2'-methyl group.

54. Medicament according to one of claims 32 to 53, where at least one nucleotide in at least one strand of the double-stranded structure is a locked nucleotide with a sugar ring which is chemically modified, preferably by a 2'-O, 4'-C-methylene bridge.

55. Medicament according to one of claims 32 to 54, where the dsRNA is bound to, associated with or surrounded by, at least one viral coat protein which originates from a virus, is derived therefrom or has been prepared synthetically.

56. Medicament according to one of claims 32 to 55, where the coat protein is derived from the polyomavirus.

57. Medicament according to one of claims 32 to 56, where the coat protein contains the polyomavirus virus protein 1 (VP1) and/or virus protein 2 (VP2).

58. Medicament according to one of claims 32 to 57, where, when a capsid or capsid-type structure is formed from the coat protein, one side faces the interior of the capsid or capsid-type structure.

59. Medicament according to one of claims 32 to 58, where one strand of the dsRNA is complementary to the primary or processed RNA transcript of the target gene.

60. Medicament according to one of claims 32 to 59, where the cell is a vertebrate cell or a human cell.

61. Medicament according to one of claims 32 to 60, where at least two dsRNAs which differ from each other are contained in the medicament, where at least segments of one strand of each dsRNA are complementary to in each case one of at least two different target genes.

62. Medicament according to claim 61, where one of the target genes is the PKR gene.

63. Active ingredient with at least one oligoribonucleotide with double-stranded structure (dsRNA) formed by two separate RNA single strands for inhibiting the expression of a given target gene, where one strand of the dsRNA has a region which is complementary to the target gene, and where the target gene is part of a phytopathogenic virus or viroid,
**characterized in that**
the complementary region has less than 25 successive nucleotide pairs.

64. Active ingredient according to claim 63, where the target gene can be expressed in eukaryotic cells.

65. Active ingredient according to claim 63 or 64, where segments of the dsRNA are in double-stranded form.

66. Active ingredient according to one of claims 63 to 65, where the ends of the dsRNA are modified in order to counteract degradation in the cell or dissociation into the single strands.

67. Active ingredient according to one of claims 63 to 66, where the cohesion of the double-stranded structure, which is caused by the complementary nucleotide pairs, is increased by at least one, preferably two, further chemical linkage(s).

68. Active ingredient according to one of claims 63 to 67, where the chemical linkage is formed by a covalent or ionic bond, a hydrogen bond, hydrophobic interactions, preferably van-der-Waals or stacking interactions, or by metal-ion coordination.

69. Active ingredient according to one of claims 63 to 68, where the chemical linkage is generated at at least one, preferably both, ends of the double-stranded structure.

70. Active ingredient according to one of claims 63 to 69, where the chemical linkage is formed by means of one or more compound groups, the compound groups preferably being poly(oxyphosphinicooxy-1,3-propanediol) and/or polyethylene glycol chains.

71. Active ingredient according to one of claims 63 to 70, where the chemical linkage is formed by purine analogs used in the double-stranded structure in place of purines.

72. Active ingredient according to one of claims 63 to 71, where the chemical linkage is formed by azabenzene units inserted into the double-stranded structure.

73. Active ingredient according to one of claims 63 to 72, where the chemical linkage is formed by branched nucleotide analogs used in the double-stranded structure in place of nucleotides.

74. Active ingredient according to one of claims 63 to 73, where at least one of the following groups is used for generating the chemical linkage: methylene blue; bifunctional groups, preferably bis(2-chloroethyl)amine; N-acetyl-N'-(p-glyoxylbenzoyl)cystamine; 4-thiouracil; psoralene.

75. Active ingredient according to one of claims 63 to 74, where the chemical linkage is formed by thiophosphoryl groups provided at the ends of the double-stranded structure.

76. Active ingredient according to one of claims 63 to 75, where the chemical linkage are triple-helix bonds provided at the ends of the double-stranded structure.

77. Active ingredient according to one of claims 63 to 76, where at least one 2'-hydroxyl group of the nucleotides of the dsRNA in the double-stranded structure is replaced by a chemical group, preferably a 2'-amino or a 2'-methyl group.

78. Active ingredient according to one of claims 63 to 77, where at least one nucleotides at least one strand of the double-stranded structure is a locked nucleotide with a sugar ring which is chemically modified, preferably by a 2'-O, 4'-C-methylene bridge.

79. Active ingredient according to one of claims 63 to 78, where one strand of the dsRNA is complementary to the primary or processed RNA transcript of the target gene.

80. Active ingredient according to one of claims 63 to 79, where at least two dsRNAs which differ from each other are contained in the active ingredient, where at least segments of one strand of each dsRNA are complementary to in each case one of at least two different target genes.

81. Use of an oligoribonucleotide with double-stranded structure (dsRNA) formed by two separate RNA single strandsfor preparing a medicament or active ingredient for inhibiting the expression of a given target gene, where one strand of the dsRNA has a region which is complementary to the target gene,
**characterized in that**
the complementary region has less than 25 successive nucleotide pairs.

82. Use according to claim 81, where the dsENA is enclosed by micellar structures, preferably by liposomes.

83. Use according to either of claims 81 or 82, where the dsRNA is enclosed by natural viral capsids or by chemically or enzymatically produced artificial capsids or structures derived therefrom.

84. Use according to one of claims 81 to 83, where the target gene can be expressed in eukaryotic cells.

85. Use according to one of claims 81 to 84, where the target gene is selected from the following group: oncogene, cytokin gene, Id-protein gene, development gene, prion gene.

86. Use according to one of claims 81 to 85, where the target gene can be expressed in pathogenic organisms, preferably in plasmodia.

87. Use according to one of claims 81 to 86, where the target gene is part of a virus or viroid.

88. Use according to claim 87, where the virus is a virus or viroid which is pathogenic for humans.

89. Use according to claim 87, where the virus or viroid is a virus or viroid which is pathogenic for animals or phytopathogenic.

90. Use according to one of claims 81 to 89, where segments of the dsRNA are in double-stranded form.

91. Use according to one of claims 81 to 90, where the ends of the dsRNA are modified in order to counteract degradation in the cell or dissociation into the single strands.

92. Use according to one of claims 81 to 91, where the cohesion of the double-stranded structure, which is caused by the complementary nucleotide pairs, is increased by at least one, preferably two, further chemical linkage(s).

93. Use according to one of claims 81 to 92, where the chemical linkage is formed by a covalent or ionic bond, a hydrogen bond, hydrophobic interactions, preferably van-der-Waals or stacking interactions, or by metal-ion coordination.

94. Use according to one of claims 81 to 93, where the chemical linkage is generated at at least one, preferably both, ends of the double-stranded structure.

95. Use according to one of claims 81 to 94, where the chemical linkage is formed by means of one or more compound groups, the compound groups preferably being poly(oxyphosphinicooxy-1,3-propanediol) and/or polyethylene glycol chains.

96. Use according to one of claims 81 to 95, where the chemical linkage is formed by purine analogs used in the double-stranded structure in place of purines.

97. Use according to one of claims 81 to 96, where the chemical linkage is formed by azabenzene units introduced into the double-stranded structure.

98. Use according to one of claims 81 to 97, where the chemical linkage is formed by branched nucleotide analogs used in the double-stranded structure in place of nucleotides.

99. Use according to one of claims 81 to 98, where at least one of the following groups is used for generating the chemical linkage: methylene blue; bifunctional groups, preferably bis(2-chloroethyl)amine; N-acetyl-N'-(p-glyoxylbenzoyl)-cystamine; 4-thiouracil; psoralene.

100. Use according to one of claims 81 to 99, where the chemical linkage is formed by thiophosphoryl groups attached to the ends of the double-stranded structure.

101. Use according to one of claims 81 to 100, where the chemical linkage at the ends of the double-stranded structure is formed by triple-helix bonds.

102. Use according to one of claims 81 to 101, where at least one 2'-hydroxyl group of the nucleotides of the dsRNA in the double-stranded structure is replaced by a chemical group, preferably a 2'-amino or a 2'-methyl group.

103. Use according to one of claims 81 to 102, where at least one nucleotide in at least one strand of the double-stranded structure is a locked nucleotide with a sugar ring which is chemically modified, preferably by a 2'-O, 4'-C-methylene bridge.

104. Use according to one of claims 81 to 103, where the dsRNA is bound to, associated with or surrounded by, at least one viral coat protein which originates from a virus, is derived therefrom or has been prepared synthetically.

105. Use according to one of claims 81 to 104, where the coat protein is derived from polyomavirus.

106. Use according to one of claims 81 to 105, where the coat protein contains the polyomavirus virus protein 1 (VP1) and/or virus protein 2 (VP2).

107. Use according to one of claims 81 to 106, where, when a capsid or capsid-type structure is formed from the coat protein, one side faces the interior of the capsid or capsid-type structure.

108. Use according to one of claims 81 to 107, where one strand of the dsRNA is complementary to the primary or processed RNA transcript of the target gene.

109. Use according to one of claims 81 to 108, where the cell is a vertebrate cell or a human cell.

110. Use according to one of claims 81 to 109, where at least two dsRNAs which differ from each other are used, where at least segments of one strand of each dsRNA are complementary to in each case one of at least two different target genes.

111. Use according to claim 110, where one of the target genes is the PKR gene.

112. Use according to one of claims 81 to 111, where the medicament is injectable into the bloodstream or into the interstitium of the organism to undergo therapy.

113. Use according to one of claims 81 to 112, where the dsRNA is taken up into bacteria or microorganisms.

114. Use of a vector for coding at least one oligoribonucleotide with double-stranded structure (dsBNA) formed by two separate RNA single strands for preparing a medicament or active ingredient for inhibiting the expression of a given target gene, where one strand of the dsRNA has a region which is complementary to the target gene,
**characterized in that**
the complementary region has less than 25 successive nucleotide pairs.

115. Use according to claim 114, where the target gene can be expressed in eukaryotic cells.

116. Use according to claim 114 or 115, where the target gene is selected from the following group: oncogene, cytokin gene, Id-protein gene, development gene, prion gene.

117. Use according to one of claims 114 to 116, where the target gene can be expressed in pathogenic organisms, preferably in plasmodia.

118. Use according to one of claims 114 to 117, where the target gene is part of a virus or viroid.

119. Use according to claim 118, where the virus is a virus or viroid which is pathogenic for humans.

120. Use according to claim 118, where the virus or viroid is a virus or viroid which is pathogenic for animals or phytopathogenic.

121. Use according to one of claims 114 to 120, where segments of the dsRNA are in double-stranded form.

122. Use according to one of claims 114 to 121, where one strand of the dsRNA is complementary to the primary or processed RNA transcript of the target gene.

123. Use according to one of claims 114 to 122, where the cell is a vertebrate cell or a human cell.

124. Use according to one of claims 114 to 123, where at least two dsRNAs which differ from each other are used, where at least segments of one strand of each dsRNA are complementary to in each case one of at least two different target genes.

125. Use according to claim 125, where one of the target genes is the PKR gene.

## Revendications

1. Procédé d'inhibition de l'expression d'un gène cible donné dans une cellule in vitro, dans lequel un oligoribonucléotide d'une structure double brin (ARNdb) formée de deux ARN simple brin séparés est introduit dans la cellule, un brin de l'ARNdb présentant une région complémentaire du gène cible,
**caractérisé en ce que**
la région complémentaire présente moins de 25 paires de nucléotides successives.

2. Procédé selon la revendication 1, dans lequel l'ARNdb est incorporé dans des structures micellaires, de préférence dans des liposomes.

3. Procédé selon l'une des revendications précédentes, dans lequel l'ARNdb est incorporé dans des capsides naturelles virales ou dans des capsides artificielles, préparées par voie chimique ou enzymatique, ou dans des structures en dérivant.

4. Procédé selon l'une des revendications précédentes, dans lequel le gène cible est exprimé dans des cellules eucaryotes.

5. Procédé selon l'une des revendications précédentes, dans lequel le gène cible est sélectionné parmi le groupe suivant: oncogène, gène de cytokine, gène d'Id-protéine, gène de développement, gène de prion.

6. Procédé selon l'une des revendications précédentes, dans lequel le gène cible est exprimé dans des organismes pathogènes, de préférence dans des plasmodies.

7. Procédé selon l'une des revendications précédentes, dans lequel le gêne cible est une composante d'un virus ou d'un viroïde.

8. Procédé selon la revendication 7, dans lequel le virus est un virus ou un viroïde pathogène humain.

9. Procédé selon la revendication 7, dans lequel le virus ou le viroïde est un virus ou un viroïde pathogène animal ou végétal.

10. Procédé selon l'une des revendications précédentes, dans lequel l'ARNdb est réalisé en double brin sur certaines portions ou segments.

11. Procédé selon l'une des revendications précédentes, dans lequel les extrémités de l'ARNdb sont modifiées pour contrer une dégradation dans la cellule ou une dissociation en simples brins.

12. Procédé selon l'une des revendications précédentes, dans lequel la cohésion de la structure double brin, occasionnée par les paires de nucléotides complémentaires, est renforcée par au moins une, de préférence deux, autre(s) liaison(s) chimique(s).

13. Procédé selon l'une des revendications précédentes, dans lequel la liaison chimique est formée par une liaison covalente ou ionique, une liaison par pontage d'hydrogène, des interactions hydrophobes, de préférence des interactions Van der Waals ou d'empilement, ou par une coordination ionique métallique.

14. Procédé selon l'une des revendications précédentes, dans lequel la liaison chimique est réalisée au niveau d'au moins une, de préférence au niveau des deux extrémités de la structure double brin.

15. Procédé selon l'une des revendications précédentes, dans lequel la liaison chimique est formée au moyen d'un ou de plusieurs groupes de liaison, les groupes de liaison étant de préférence des chaînes poly-(oxyphosphinico-oxy-1,3-propanediol)- et/ou polyéthylèneglycol.

16. Procédé selon l'une des revendications précédentes, dans lequel la liaison chimique est formée par des analogues de purine utilisés à la place de la purine dans la structure double brin.

17. Procédé selon l'une des revendications précédentes, dans lequel la liaison chimique est formée par des motifs azabenzène introduits dans la structure double brin.

18. Procédé selon l'une des revendications précédentes, dans lequel la liaison chimique est formée par des analogues nucléotidiques ramifiés utilisés à la place de nucléotides dans la structure double brin.

19. Procédé selon l'une des revendications précédentes, dans lequel on utilise au moins un des groupes suivants pour former la liaison chimique : bleu de méthylène ; groupes bifonctionnels, de préférence une bis-(2-chloroéthyl)amine ; N-acétyl-N'-(p-glyoxyl-benzoyl)-cystamine; 4-thiouracile ; psoralène.

20. Procédé selon l'une des revendications précédentes, dans lequel la liaison chimique est formée par des groupes thiophosphoryle appliqués au niveau des extrémités de la structure double brin.

21. Procédé selon l'une des revendications précédentes, dans lequel la liaison chimique est formée au niveau des extrémités de la structure double brin par des liaisons en triple hélice.

22. Procédé selon l'une des revendications précédentes, dans lequel au moins un groupe 2'-hydroxyle des nucléotides de l'ARNdb est remplacé dans la structure double brin par un groupe chimique, de préférence un groupe 2'-amino- ou 2'-méthyle.

23. Procédé selon l'une des revendications précédentes, dans lequel au moins un nucléotide d'au moins un brin de la structure double brin est un "nucléotide fermé" présentant un cycle sucre modifié chimiquement, de préférence par un pont 2'-O,4'-C-méthylène.

24. Procédé selon l'une des revendications précédentes, dans lequel l'ARNdb est lié à, associé à ou entouré par au moins une protéine enveloppe virale provenant d'un virus, dérivée de celui-ci ou fabriquée par voie synthétique.

25. Procédé selon l'une des revendications précédentes, dans lequel la protéine enveloppe est dérivée d'un polyomavirus.

26. Procédé selon l'une des revendications précédentes, dans lequel la protéine enveloppe contient la protéine virale 1 (VP1) et/ou la protéine virale 2 (VP2) du polyomavirus.

27. Procédé selon l'une des revendications précédentes, dans lequel, lors de la formation d'une capside ou d'une structure de type capside à partir de la protéine enveloppe, un côté est tourné vers l'intérieur de la capside ou de la structure de type capside.

28. Procédé selon l'une des revendications précédentes, dans lequel un brin de l'ARNdb est complémentaire du transcript d'ARN primaire ou traité du gène cible.

29. Procédé selon l'une des revendications précédentes, dans lequel la cellule est une cellule de vertébré ou une cellule humaine.

30. Procédé selon l'une des revendications précédentes, dans lequel au moins deux ARNdb différents l'un de l'autre sont introduits dans la cellule, un brin de chaque ARNdb étant complémentaire au moins sur certaines portions ou segments de respectivement un d'au moins deux gènes cibles différents.

31. Procédé selon l'une des revendications précédentes, dans lequel un des gènes cible est le gène PKR.

32. Médicament comportant au moins un oligoribonucléotide d'une structure double brin (ARNdb) formée de deux ARN simple brin séparés, destiné à inhiber l'expression d'un gène cible donné, un brin de l'ARNdb présentant une région complémentaire du gène cible,
**caractérisé en ce que**
la région complémentaire présente moins de 25 paires de nucléotides successives.

33. Médicament selon la revendication 32, dans lequel l'ARNdb est empaqueté dans des structures micellaires, de préférence des liposomes.

34. Médicament selon l'une des revendications 32 ou 33, dans lequel l'ARNdb est incorporé dans des capsides naturelles virales ou dans des capsides artificielles, préparées par voie chimique ou enzymatique, ou dans des structures en dérivant.

35. Médicament selon l'une des revendications 32 à 34, dans lequel le gène cible peut être exprimé dans des cellules eucaryotes.

36. Médicament selon l'uns des revendications 32 à 35, dans lequel le gène cible est sélectionné parmi le groupe suivant : oncogène, gène de cytokine, gène d'Id-protéine, gène de développement, gène de prion.

37. Médicament selon l'une des revendications 32 à 36, dans lequel le gène cible peut être exprimé dans des organismes pathogènes, de préférence dans des plasmodies.

38. Médicament selon l'une des revendications 32 à 37, dans lequel le gène cible est une composante d'un virus ou d'un viroïde.

39. Médicament selon la revendication 38, dans lequel le virus est un virus ou un viroïde pathogène humain.

40. Médicament selon la revendication 38, dans lequel le virus ou le viroïde est un virus ou un viroïde pathogène animal.

41. Médicament selon l'une des revendications 32 à 40, dans lequel l'ARNdb est réalisé en double brin sur certaines portions ou segments.

42. Médicament selon l'une des revendications 32 à 40, dans lequel les extrémités de l'ARNdb sont modifiées pour contrer une dégradation dans la cellule ou une dissociation en simples brins.

43. Médicament selon l'une des revendications 32 à 42, dans lequel la cohésion de la structure double brin, occasionnée par les paires de nucléotides complémentaires, est renforcée par au moins une, de préférence deux, autre(s) liaison(s) chimique(s).

44. Médicament selon l'une des revendications 32 à 43, dans lequel la liaison chimique est formée par une liaison covalente ou ionique, une liaison par pontage d'hydrogène, des interactions hydrophobes, de préférence des interactions Van der Waals ou d'empilement, ou par une coordination ionique métallique.

45. Médicament selon l'une des revendications 32 à 44, dans lequel la liaison chimique est réalisée au niveau d'au moins une, de préférence au niveau des deux, extrémités de la structure double brin.

46. Médicament selon l'une des revendications 32 à 45, dans lequel la liaison chimique est formée au moyen d'un ou de plusieurs groupes de liaison, les groupes de liaison étant de préférence des chaînes poly-(oxyphosphinico-oxy-1,3-propanediol)- et/ou polyéthylèneglycol.

47. Médicament selon l'une des revendications 32 à 46, dans lequel la liaison chimique est formée par des analogues de purine utilisés à la place de la purine dans la structure double brin.

48. Médicament selon l'une des revendications 32 à 47, dans lequel la liaison chimique est formée par des motifs azabenzène introduits dans la structure double brin.

49. Médicament selon l'une des revendications 32 à 48, dans lequel la liaison chimique est formée par des analogues nucléotidiques ramifiés utilisés à la place de nucléotides dans la structure double brin.

50. Médicament selon l'une des revendications 32 à 49, dans lequel on utilise au moins un des groupes suivants pour former la liaison chimique : bleu de méthylène ; groupes bifonctionnels, de préférence une bis-(2-chloroéthyl)amine ; N-acétyl-N'-(p-glyoxyl-benzoyl)-cystamine ; 4-thiouracile; psoralène.

51. Médicament selon l'une des revendications 32 à 50, dans lequel la liaison chimique est formée par des groupes thiophosphoryle prévus au niveau des extrémités de la structure double brin.

52. Médicament selon l'une des revendications 32 à 51, dans lequel la liaison chimique est constituée par des liaisons en triple hélice au niveau des extrémités de la structure double brin.

53. Médicament selon l'une des revendications 32 à 52, dans lequel au moins un groupe 2'-hydroxyle des nucléotides de l'ARNdb est remplacé dans la structure double brin par un groupe chimique, de préférence un groupe 2'-amino- ou 2'-méthyle.

54. Médicament selon l'une des revendications 32 à 53, dans lequel au moins un nucléotide d'au moins un brin de la structure double brin est un "nucléotide fermé" présentant un cycle sucre modifié chimiquement, de préférence par un pont 2'-O,4'-C-méthylène.

55. Médicament selon l'une des revendications 32 à 54, dans lequel l'ARNdb est lié à, associé à ou entouré par au moins une protéine enveloppe virale provenant d'un virus, dérivée de celui-ci ou fabriquée par voie synthétique.

56. Médicament selon l'une des revendications 32 à 55, dans lequel la protéine enveloppe est dérivée d'un polyomavirus.

57. Médicament selon l'une des revendications 32 à 56, dans lequel la protéine enveloppe contient la protéine virale 1 (VP1) et/ou la protéine virale 2 (VP2) du polyomavirus.

58. Médicament selon l'une des revendications 32 à 57, dans lequel, lors de la formation d'une capside ou d'une structure de type capside à partir de la protéine enveloppe, un côté est tourné vers l'intérieur de la capside ou de la structure de type capside.

59. Médicament selon l'une des revendications 32 à 58, dans lequel un brin de l'ARNdb est complémentaire du transcript d'ARN primaire ou traité du gène cible.

60. Médicament selon l'une des revendications 32 à 59, dans lequel la cellule est une cellule de vertébré ou une cellule humaine.

61. Médicament selon l'une des revendications 32 à 60, dans lequel sont contenus au moins deux ARNdb différents l'un de l'autre, un brin de chaque ARNdb étant complémentaire au moins sur certaines portions ou segments de respectivement un d'au moins deux gènes cibles différents.

62. Médicament selon la revendication 61, dans lequel un des gènes cible est le gène PKR.

63. Principe actif comportant au moins un oligoribonucléotide d'une structure double brin (ARNdb) formée de deux ARN simple brin séparés, destiné à inhiber l'expression d'un gène cible donné, un brin de l'ARNdb présentant une région complémentaire du gène cible, et dans lequel le gène cible est une composante d'un virus ou d'un viroïde pathogène végétal,
**caractérisé en ce que**
la région complémentaire présente moins de 25 paires de nucléotides successives.

64. Principe actif selon la revendication 63, dans lequel le gène cible peut être exprimé dans des cellules eucaryotes.

65. Principe actif selon la revendication 63 ou 64, dans lequel l'ARNdb est réalisé en double brin sur certaines portions ou segments.

66. Principe actif selon l'une des revendications 63 à 65, dans lequel les extrémités de l'ARNdb sont modifiées pour contrer une dégradation dans la cellule ou une dissociation en simples brins.

67. Principe actif selon l'une des revendications 63 à 66, dans lequel la cohésion de la structure double brin, occasionnée par les paires de nucléotides complémentaires, est renforcée par au moins une, de préférence deux, autre(s) liaison(s) chimique(s).

68. Principe actif selon l'une des revendications 63 à 67, dans lequel la liaison chimique est formée par une liaison covalente ou ionique, une liaison par pontage d'hydrogène, des interactions hydrophobes, de préférence des interactions Van der Waals ou d'empilement, ou par une coordination ionique métallique.

69. Principe actif selon l'une des revendications 63 à 68, dans lequel la liaison chimique est réalisée au niveau d'au moins une, de préférence au niveau des deux, extrémités de la structure double brin.

70. Principe actif selon l'une des revendications 63 à 69, dans lequel la liaison chimique est formée au moyen d'un ou de plusieurs groupes de liaison, les groupes de liaison étant de préférence des chaînes poly-(oxyphosphinico-oxy-1,3-propanediol)- et/ou polyéthylèneglycol.

71. Principe actif selon l'une des revendications 63 à 70, dans lequel la liaison chimique est formée par des analogues de purine utilisés à la place de la purine dans la structure double brin.

72. Principe actif selon l'une des revendications 63 à 71, dans lequel la liaison chimique est formée par des motifs azabenzène introduits dans la structure double brin.

73. Principe actif selon l'une des revendications 63 à 72, dans lequel la liaison chimique est formée par des analogues nucléotidiques ramifiés utilisés à la place de nucléotides dans la structure double brin.

74. Principe actif selon l'une des revendications 63 à 73, dans lequel on utilise au moins un des groupes suivants pour former la liaison chimique : bleu de méthylène ; groupes bifonctionnels, de préférence une bis-(2-chloroéthyl)amine ; N-acétyl-N'-(p-glyoxyl-benzoyl)-cystamine ; 4-thiouracile; psoralène.

75. Principe actif selon l'une des revendications 63 à 74, dans lequel la liaison chimique est formée par des groupes thiophosphoryle prévus au niveau des extrémités de la structure double brin.

76. Principe actif selon l'une des revendications 63 à 75, dans lequel la liaison chimique est constituée par des liaisons en triple hélice au niveau des extrémités de la structure double brin.

77. Principe actif selon l'une des revendications 63 à 76, dans lequel au moins un groupe 2'-hydroxyle des nucléotides de l'ARNdb est remplacé dans la structure double brin par un groupe chimique, de préférence un groupe 2'-amino- ou 2'-méthyle.

78. Principe actif selon l'une des revendications 63 à 77, dans lequel au moins un nucléotide d'au moins un brin de la structure double brin est un "nucléotide fermé" présentant un cycle sucre modifié chimiquement, de préférence par un pont 2'-O,4'-C-méthylène.

79. Principe actif selon l'une des revendications 63 à 78, dans lequel un brin de l'ARNdb est complémentaire du transcript d'ARN primaire ou traité du gène cible.

80. Principe actif selon l'une des revendications 63 à 79, dans lequel sont contenus au moins deux ARNdb différents l'un de l'autre, un brin de chaque ARNdb étant complémentaire au moins sur certaines portions ou segments de respectivement un d'au moins deux gènes cibles différents.

81. Utilisation d'un oligoribonucléotide d'une structure double brin (ARNdb) formée de deux ARN simple brin séparés pour préparer un médicament ou un principe actif destiné à inhiber l'expression d'un gène cible donné, un brin de l'ARNdb présentant une région complémentaire du gène cible,
**caractérisée en ce que**
la région complémentaire présente moins de 25 paires de nucléotides successives.

82. Utilisation selon la revendication 81, dans laquelle l'ARNdb est empaqueté dans des structures micellaires, de préférence des liposomes.

83. Utilisation selon l'une des revendications 81 ou 82, dans laquelle l'ARNdb est incorporé dans des capsides naturelles virales ou dans des capsides artificielles, préparées par voie chimique ou enzymatique, ou dans des structures en dérivant.

84. Utilisation selon l'une des revendications 81 à 83, dans laquelle le gène cible peut être exprimé dans des cellules eucaryotes.

85. Utilisation selon l'une des revendications 81 à 84, dans laquelle le gène cible est sélectionné parmi le groupe suivant : oncogène, gène de cytokine, gène d'Id-protéine, gène de développement, gène de prion.

86. Utilisation selon l'une des revendications 81 à 85, dans laquelle le gène cible peut être exprimé dans des organismes pathogènes, de préférence dans des plasmodies.

87. Utilisation selon l'une des revendications 81 à 86, dans laquelle le gène cible est une composante d'un virus ou d'un viroïde.

88. Utilisation selon la revendication 87, dans laquelle le virus est un virus ou un viroïde pathogène humain.

89. Utilisation selon la revendication 87, dans laquelle le virus ou le viroïde est un virus ou un viroïde pathogène animal ou végétal.

90. Utilisation selon l'une des revendications 81 à 89, dans laquelle l'ARNdb est réalisé à double brin sur certaines portions ou segments.

91. Utilisation selon l'une des revendications 81 à 90, dans laquelle les extrémités de l'ARNdb sont modifiées pour contrer une dégradation dans la cellule ou une dissociation en simples brins.

92. Utilisation selon l'une des revendications 81 à 91, dans laquelle la cohésion de la structure double brin, occasionnée par les paires de nucléotides complémentaires, est renforcée par au moins une, de préférence deux, autre(s) liaison(s) chimique(s).

93. Utilisation selon l'une des revendications 81 à 92, dans laquelle a liaison chimique est formée par une liaison covalente ou ionique, une liaison par pontage d'hydrogène, des interactions hydrophobes, de préférence des interactions Van der Waals ou d'empilement, ou par une coordination ionique métallique.

94. Utilisation selon l'une des revendications 81 à 93, dans laquelle la liaison chimique est réalisée au niveau d'au moins une, de préférence au niveau des deux, extrémités de la structure double brin.

95. Utilisation selon l'une des revendications 81 à 94, dans laquelle la liaison chimique est formée au moyen d'un ou de plusieurs groupes de liaison, les groupes de liaison étant de préférence des chaînes poly-(oxyphosphinico-oxy-1,3-propanediol)- et/ou polyéthylèneglycol.

96. Utilisation selon l'une des revendications 81 à 95, dans laquelle la liaison chimique est formée par des analogues de purine utilisés à la place de la purine dans la structure double brin.

97. Utilisation selon l'une des revendications 81 à 96, dans laquelle la liaison chimique est formée par des motifs azabenzène introduits dans la structure double brin.

98. Utilisation selon l'une des revendications 81 à 97, dans laquelle la liaison chimique est formée par des analogues nucléotidiques ramifiés utilisés à la place de nucléotides dans la structure double brin.

99. Utilisation selon l'une des revendications 81 à 98, dans laquelle on utilise au moins un des groupes suivants pour former la liaison chimique : bleu de méthylène ; groupes bifonctionnels, de préférence une bis-(2-chloroéthyl)amine ; N-acétyl-N'-(p-glyoxyl-benzoyl)-cystamine ; 4-thiouracile ; psoralène.

100. Utilisation selon l'une des revendications 81 à 99, dans laquelle la liaison chimique est formée par des groupes thiophosphoryle appliqués au niveau des extrémités de la structure double brin.

101. Utilisation selon l'une des revendications 81 à 100, dans laquelle la liaison chimique est formée au niveau des extrémités de la structure double brin par des liaisons en triple hélice.

102. Utilisation selon l'une des revendications 81 à 101, dans laquelle au moins un groupe 2'-hydroxyle des nucléotides de l'ARNdb est remplacé dans la structure double brin par un groupe chimique, de préférence un groupe 2'-amino- ou 2'-méthyle.

103. Utilisation selon l'une des revendications 81 à 102, dans laquelle au moins un nucléotide d'au moins un brin de la structure double brin est un "nucléotide fermé" présentant un cycle sucre modifié chimiquement, de préférence par un pont 2'-O,4'-C-méthylène.

104. Utilisation selon l'une des revendications 81 à 103, dans laquelle l'ARNdb est lié à, associé à ou entouré par au moins une protéine enveloppe virale provenant d'un virus, dérivée de celui-ci ou fabriquée par voie synthétique.

105. Utilisation selon l'une des revendications 81 à 104, dans laquelle la protéine enveloppe est dérivée d'un polyomavirus.

106. Utilisation selon l'une des revendications 81 à 105, dans laquelle la protéine enveloppe contient la protéine virale 1 (VP1) et/ou la protéine virale 2 (VP2) du polyomavirus.

107. Utilisation selon l'une des revendications 81 à 106, dans laquelle lors de la formation d'une capside ou d'une structure de type capside à partir de la protéine enveloppe, un côté est tourné vers l'intérieur de la capside ou de la structure de type capside.

108. Utilisation selon l'une des revendications 81 à 107, dans laquelle un brin de l'ARNdb est complémentaire du transcript d'ARN primaire ou traité du gène cible.

109. Utilisation selon l'une des revendications 81 à 108, dans laquelle la cellule est une cellule de vertébré ou une cellule humaine.

110. Utilisation selon l'une des revendications 81 à 109, dans laquelle au moins deux ARNdb différents l'un de l'autre sont utilisés, un brin de chaque ARNdb étant complémentaire au moins sur certaines portions ou segments de respectivement un d'au moins deux gènes cibles différents.

111. Utilisation selon la revendication 110, dans laquelle un des gènes cible est le gène PKR.

112. Utilisation selon l'une des revendications 81 à 111, dans laquelle le médicament peut être injecté dans le flux sanguin ou dans l'interstitium de l'organisme sur lequel est réalisée la thérapie.

113. Utilisation selon l'une des revendications 81 à 112, dans laquelle l'ARNdb est recueilli dans des bactéries ou des microorganismes.

114. Utilisation d'un vecteur de codage d'au moins un oligoribonucléotide d'une structure double brin (ARNdb) formée de deux ARN simple brin séparés pour fabriquer un médicament ou un principe actif destiné à inhiber l'expression d'un gène cible donné, un brin de l'ARNdb présentant une région complémentaire d'un gène cible,
**caractérisée en ce que**
la région complémentaire présente moins de 25 paires de nucléotides successives.

115. Utilisation selon la revendication 114, dans laquelle le gène cible peut être exprimé dans des cellules eucaryotes.

116. Utilisation selon la revendication 114 ou 115, dans laquelle le gène cible est sélectionné parmi le groupe suivant : oncogène, gène de cytokine, gène d'Id-protéine, gène de développement, gène de prion.

117. Utilisation selon l'une des revendications 114 à 116, dans laquelle le gène cible peut être exprimé dans des organismes pathogènes, de préférence dans des plasmodies.

118. Utilisation selon l'une des revendications 114 à 117, dans laquelle le gène cible est une composante d'un virus ou d'un viroïde.

119. Utilisation selon la revendication 118, dans laquelle le virus est un virus ou un viroïde pathogène humain.

120. Utilisation selon la revendication 118, dans laquelle le virus ou le viroïde est un virus ou un viroïde pathogène animal ou végétal.

121. Utilisation selon l'une des revendications 114 à 120, dans laquelle l'ARNdb est réalisé en double brin sur certaines portions ou segments.

122. Utilisation selon l'une des revendications 114 à 121, dans laquelle un brin de l'ARNdb est complémentaire du transcript d'ARN primaire ou traité du gène cible.

123. Utilisation selon l'une des revendications 114 à 122, dans laquelle la cellule est une cellule de vertébré ou une cellule humaine.

124. Utilisation selon l'une des revendications 114 à 123, dans laquelle au moins deux ARNdb différents l'un de l'autre sont utilisés, un brin de chaque ARNdb étant complémentaire au moins sur certaines portions ou segments de respectivement un d'au moins deux gènes cibles différents.

125. Utilisation selon la revendication 124, dans laquelle un des gènes cible est le gène PKR.


### SEQUENZ PROTOKOLL

<110> Kreutzer Dr., Roland
   Limmer Dr., Stephan
<120> Verfahren und Medikament zur Hemmung der Expression eines vorgegebenen Gens
<130> 400968
<140>
<141>
<150> 199 03 713.2
<151> 1999-01-30
<150> 199 56 568.6
<151> 1999-11-24
<160> 8
<170> Patentin Ver. 2.1
<210> 1
<211> 45
<212> DNA
<213> Künstliche Sequenz
<220>
<223> Beschreibung der künstlichen Sequenz: EcoRI-Schnittstelle, T7-RNA-Polymerasepromotor
<400> 1
   ggaattctaa tacgactcac tatagggcga tcagatctct agaag                45
<210> 2
<211> 50
<212> DNA
<213> Künstliche Sequenz
<220>
<223> Beschreibung der künstlichen Sequenz:
   BamHI-Schnittstelle, SP6-RNA-Polymerasepromotor
<400> 2
   gggatccatt taggtgacac tatagaatac ccatgatcgc gtagtcgata           50
<210> 3
<211> 340
<212> RNA
<213> Künstliche Sequenz
<220>
<223> Beschreibung der künstlichen Sequenz: RNA, die einer Sequenz aus der "positive control DNA" des HeLaScribe Nuclear Extract in vitro Transkriptionskits der Firma Promega entspricht
<400> 3
   ucagaucucu agaagcuuua augcgguagu uuaucacagu uaaauugcua acgcagucag 60
   gcaccgugua ugaaaucuaa caaugcgcuc aucgucaucc ucggcaccgu cacccuggau 120
   gcuguaggca uaggcuuggu uaugccggua cugccgggcc ucuugcggga uaucguccau 180
   uccgacagca ucgccaguca cuauggcgug cugcuagcgc uauaugcguu gaugcaauuu 240
   cuaugcgcac ccguucucgg agcacugucc gaccgcuuug gccgccgccc aguccugcuc 300
   gcuucgcuac uuggagccac uaucgacuac gcgaucaugg                       340
<210> 4
<211> 363
<212> DNA
<213> Künstliche Sequenz
<220>
<223> Beschreibung der künstlichen Sequenz: DNA, die einer Sequenz aus der "positive control DNA" des HeLaScribe Nuclear Extract in vitro Transkriptionskits der Firma Promega entspricht
<400> 4
   tcagatctct agaagcttta atgcggtagt ttatcacagt taaattgcta acgcagtcag 60
   gcaccgtgta tgaaatctaa caatgcgctc atcgtcatcc tcggcaccgt caccctggat 120
   gctgtaggca taggcttggt tatgccggta ctgccgggcc tcttgcggga tatcgtccat 180
   tccgacagca tcgccagtca ctatggcgtg ctgctagcgc tatatgcgtt gatgcaattt 240
   ctatgcgcac ccgttctcgg agcactgtcc gaccgctttg gccgccgccc agtcctgctc 300
   gcttcgctac ttggagccac tatcgactac gcgatcatgg cgaccacacc cgtcctgtgg 360
   atc                                                               363
<210> 5
<211> 315
<212> RNA
<213> Künstliche Sequenz
<220>
<223> Beschreibung der künstlichen Sequenz: Sequenz aus dem YFP-Gen
<400> 5
   auggugagca agggcgagga gcuguucacc gggguggugc ccauccuggu cgagcuggac 60
   ggcgacguaa acggccacaa guucagcgug uccggcgagg gcgagggcga ugccaccuac 120
   ggcaagcuga cccugaaguu caucugcacc accggcaagc ugcccgugcc cuggcccacc 180
   cucgugacca cccugaccua cggcgugcag ugcuucagcc gcuaccccga ccacaugaag 240
   cagcacgacu ucuucaaguc cgccaugccc gaaggcuacg uccaggagcg caccaucuuc 300
   uucaaggacg acggc                                                  315
<210> 6
<211> 52
<212> DNA
<213> Künstliche Sequenz
<220>
<223> Beschreibung der künstlichen Sequenz: EcoRI-Schnittstelle, T7-RNA-Polymerasepromotor, komplementärer Bereich zum YFP-Gen
<400> 6
   ggaattctaa tacgactcac tatagggcga atggtgagca agggcgagga gc 52
<210> 7
<211> 53
<212> DNA
<213> Künstliche Sequenz
<220>
<223> Beschreibung der künstlichen Sequenz: BamHI-Schnittstelle, SP6-RNA-Polymerasepromotor, komplementärer Bereich zum YFP-Gen
<400> 7
   gggatccatt taggtgacac tatagaatac gccgtcgtcc ttgaagaaga tgg 53
<210> 8
<211> 21
<212> RNA
<213> Künstliche Sequenz
<220>
<223> Beschreibung der künstlichen Sequenz: RMA, die einer Sequenz aus dem YFP-Gen entspricht
<400> 8
   ucgagcugga cggcgacgua a 21